(19) Europäisches Patentamt
European Patent Office
Office européen des brevets

(11) EP 1 295 863 A1

(12) EUROPEAN PATENT APPLICATION

(43) Date of publication:
**26.03.2003 Bulletin 2003/13**

(51) Int Cl.[7]: **C07C 69/76**, C09K 19/20, C09K 19/30

(21) Application number: **01810929.8**

(22) Date of filing: **24.09.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Rolic AG**
**6301 Zug (CH)**

(72) Inventors:
- **Peglow, Thomas**
**79539 Lörrach (DE)**
- **Benecke, Carsten**
**79576 Weil am Rhein (DE)**
- **Cherkaoui, Zoubair Mohammed**
**4123 Allschwil (CH)**

(74) Representative:
**Liebetanz, Michael, Dipl.-Phys. et al**
**Isler & Pedrazzini AG,**
**Patentanwälte,**
**Postfach 6940**
**8023 Zürich (CH)**

# (54) Liquid crystalline "laterally polymerizable" compounds

(57) Chiral or achiral compounds of formula (I):

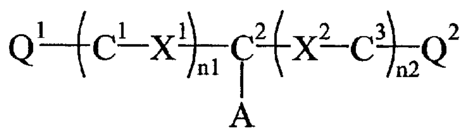

I

wherein $-(C^1-X^1)_{n1}-C^2-(X^2-C^3)_{n2}-$ is a rod-like core formed by a substantially linear arrangement of the residues represented by the symbols and A includes a polymerizable group, as well as LCP networks thereof.

EP 1 295 863 A1

## Description

The invention relates to new liquid crystalline compounds, mixtures of those compounds and their application in optical devices. More particularly, it relates to the use of a component of a polymerizable liquid crystalline mixture in the production of orientated liquid crystalline polymers; compounds used as components in polymerizable liquid crystalline mixtures; liquid crystalline mixtures comprising these components; liquid crystalline polymers and networks prepared from such components; and liquid crystalline devices comprising those compounds.

Liquid crystal polymers (LCPs) and/or liquid crystal networks are used in the manufacture of optical components such as waveguides, optical gratings, filters, retarders, rotators, piezoelectric cells and non-linear optical cells and films. The choice of LCP for use in any one of the aforementioned optical components depends upon its associated optical properties such as the optical anisotropy, refractive index, transparency and dispersion. Optical filters, for example, contain LCPs having a large anisotropy ($\Delta n$) and a low dispersion ($n = f(\lambda)$).

LCPs are manufactured by orientating a layer of a polymerizable liquid crystal single compound or mixture and cross-linking the mesogenic layer to form a liquid crystal polymer (LCP) network. Polymerizable LC compounds used in the manufacture of the LCPs need to be chemically and thermally stable, stable to electromagnetic radiation, soluble in standard solvents and miscible with other LC components, and to exhibit liquid crystalline properties over the range 25 to 80 °C, more advantageously 25 to 150 °C. The configuration imposed by an orientation layer on the polymerizable LC single compound or mixture becomes fixed or frozen into the LCP network formed upon cross-linking. The resulting LCP films have a high viscosity and are stable to mechanical stresses, temperature and light exposure.

There is therefore a need for a liquid crystalline single compound or mixture which exhibits a broad liquid-crystalline thermal range and which can be orientated on a substrate prior to cross-linking in such a way that the orientation of the LC single compound or mixture on the substrate remains stable over the period required for manufacturing the LCP network. Components which may be used in photocrosslinkable liquid crystalline layers are particularly desirable.

In previous mesogenic polymerizable compounds the polymerizable residues are attached to the mesogenic core mainly at positions ahead its long molecular axis. However, this impedes the adjustment of the desired aforementioned properties of a LCP material by suitable substituents along the long molecular axis, as for example the induction of an anisotropic permanent dipole moment by a polar substituent. Therefore, a new architecture for obtaining LCP materials was investigated, which relies on attaching of at least one polymerizable group laterally to the mesogenic core to free a peripheral position for an optional substituent ahead the long molecular axis. In addition, and as it will be demonstrated in the following examples, it was surprisingly found that a network manufactured by cross-linking an orientated layer of a liquid crystalline laterally polymerizable single compound or a mixture still exhibits anisotropic properties after curing, at least similar to those obtained with standard LCPs. Moreover, the compounds of the present invention enable the manufacture of LCP networks which are homogeneous and conserve the long molecular axis orientation induced at the monomeric scale.

Thus, the invention provides chiral or achiral compounds of formula (I):

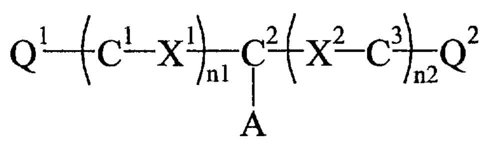

I

wherein:

$-(C^1-X^1)_{n1}-C^2-(X^2-C^3)_{n2}-$ is a rod-like core formed by a substantially linear arrangement of the residues represented by the symbols;

A — includes a polymerizable group and represents an optionally-substituted methyl group; or an optionally-substituted hydrocarbon group of 2 to 20 C-atoms, in which one or more C-atoms may be replaced by a heteroatom, in such a way that oxygen atoms are not linked to one another;

$C^1$ to $C^3$ — are in each case independently optionally-substituted nonaromatic, aromatic, carbocyclic or heterocyclic groups; preferably connected to each other at the opposite positions via the bridging groups $X^1$ and $X^2$, such as in standard mesogenic molecular architecture;

$Q^1$ — represents an optionally-substituted methyl group; or an optionally-substituted hydrocarbon group of 2 to 20 C-atoms, in which one or more C-atoms may be replaced by a heteroatom, in such a way that

oxygen atoms are not linked to one another; and which may comprise a polymerizable group;

$Q^2$ represents hydrogen; halogen; a polar group such as -CN and $-NO_2$; an optionally-substituted methyl group; an optionally-substituted hydrocarbon group of 2 to 20 C-atoms, in which one or more C-atoms may be replaced by a heteroatom, in such a way that oxygen atoms are not linked to one another; and may comprise a polymerizable group when $Q^1$ does not comprise a polymerizable group;

$X^1$ and $X^2$ each independently represent -O-, -S-, -NH-, $-N(CH_3)-$, -N=N-, -CO-C=C-, -CH(OH)-, -CO-, $-CH_2(CO)-$, -SO-, $-CH_2(SO)-$, $-SO_2-$, $-CH_2(SO_2)-$, -COO-, -OCO-, -OCO-O-, -S-CO-, -CO-S-, -SOO-, -OSO-, -SOS-, $-CH_2-CH_2-$, $-OCH_2-$, $-CH_2O-$, -CH=CH-, -C≡C- or a single bond; and

n1 and n2 are integers, each independently having a value from 1 to 4.

The term "hydrocarbon" includes straight-chain and branched alkylene, as well as saturated and unsaturated groups. Possible substituents include alkyl, aryl (thus giving an araliphatic group) and cycloalkyl, as well as amino, cyano, epoxy, halogen, hydroxy, nitro, oxo etc. Possible heteroatoms which may replace carbon atoms include nitrogen, oxygen and sulphur. In the case of nitrogen further substitution is possible with groups such as alkyl, aryl and cycloalkyl. Likewise, the terms "alkyl" and "alkylene", as used herein, includes straight-chain and branched groups, as well as saturated and unsaturated groups

To an expert in liquid crystals, the molecular architecture of compounds of formula (I) would not have been thought to be favorable for obtaining anisotropic properties after cross-linking, because it is well known that the liquid crystalline thermal range decreases as the volume of the lateral substituent increases. Thus, the dramatic increase of the lateral substituent upon curing would have been thought to cause a considerable loss of the molecular lateral registry within the orientated mesogenic bulk of the monomers. However, we have now surprisingly found that compounds of formula (I) are suitable for the formation of networks that exhibit anisotropic properties comparable to those measured at the monomeric scale.

In a first preferred embodiment of the present invention, the group A may be selected from a residue of formula (II):

$$P^1-(Sp^1)_{m1}-(Y^1)_{k1}- \qquad \text{II}$$

wherein:

$P^1$ is a polymerizable group selected from groups comprising CH2=CW-, $CH_2$=CW-COO-, $CH_2$=C(Ph)-COO-, $CH_2$=CH-COO-Ph-, $CH_2$=CW-CO-NH-, $CH_2$=C(Ph)-CONH-, $CH_2$=C(COOR')-$CH_2$-COO-, $CH_2$=CH-O-, $CH_2$=CH-OOC-, (Ph)-CH=CH-, $CH_3$-CH=N-$(CH_2)_{p1}$-, HO-, HS-, $HO(CH_2)_{p1}COO-$, $HS(CH_2)_{p1}COO-$, HWN-, HOC(O)-, $CH_2$=CH-Ph-$(O)_{p2}$,

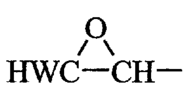 ,
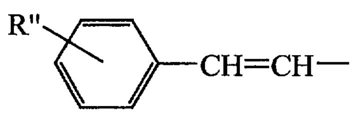 ,

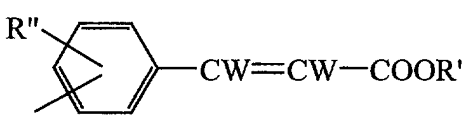 ,
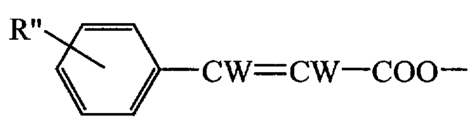 ,

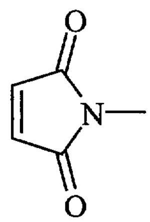 , or 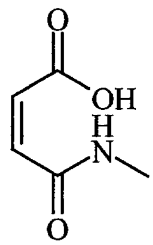 ,

wherein:

W represents H, F, Cl, Br or I or a $C_{1-5}$ alkyl group;

p1 is an integer having a value of from 1 to 9;

p2 is an integer having a value of 0 or 1,

R' represents a $C_{1-5}$ alkyl group; and

R" represents a $C_{1-5}$ alkyl group, methoxy, cyano, F, Cl, Br or I;

$Sp^1$ represents an optionally-substituted $C_{1-20}$ alkylene group, in which one or more C-atoms may be replaced by a heteroatom or/and by an optionally substituted aromatic or non-aromatic carbocyclic or heterocyclic 3- to 10-membered ring system, preferably an optionally substituted carbocyclic or heterocyclic 3-membered ring system, or/and it is optionally possible that one or more carbon-carbon single bonds are replaced by carbon-carbon double or triple bond;

m1 is an integer having a value of 0 or 1;

$Y^1$ represents -O-, -S-, -NH-, $N(CH_3)$-, -CH(OH)-, -CO-, $-CH_2(CO)-$, -SO-, $-CH_2(SO)-$, $-SO_2-$, $-CH_2(SO_2)-$, -COO-, -OCO-, -OCO-O-, -S-CO-, -CO-S-, -SOO-, -OSO-, -SOS-, $-CH_2-CH_2-$, $-OCH_2-$, $-CH_2O-$, -CH=CH-, or -C≡C-; and

k1 is an integer having a value of 0 or 1.

In relation to the residue of formula (II), the term Ph is to be understood as denoting phenylene and (Ph) as denoting phenyl.

The $C_{1-20}$ alkylene group $Sp^1$ may comprise branched or straight chain alkylene groups and may be unsubstituted, mono- or polysubstituted by F, Cl, Br, I or CN. Alternatively or in addition one or more of $CH_2$ groups present in the hydrocarbon chain may be replaced, independently, by one or more groups selected from -O-, -S-, -NH-, -N (CH3)-, -CH(OH)-, -CO-, $-CH_2(CO)-$, -SO-, $-CH_2(SO)-$, $-SO_2-$, $-CH_2(SO_2)-$, -COO-, -OCO-, -OCO-O-, -S-CO-, -CO-S-, -SOO-, -OSO-, -SOS-, -C≡C- -(CF2)q1-, or $-C(W^1)=C(W^2)-$, with the proviso that two oxygen atoms are not directly linked to each other. $W^1$ and $W^2$ each represent, independently, H, $H-(CH_2)_{q3}-$ or Cl. The integers q1 to q3 each independently represent a number of between 1 and 15.

More preferably, the group A represents a group of formula (III):

$$P^2-(Sp^2)_{m1}-Y^2- \qquad III$$

wherein:

$Y^2$ represents -O-, -CO-, -COO-, -OCO-, -C≡C-, or a single bond, especially -O-, -COO-, -OCO- or a single bond;

$Sp^2$ represents a $C_{1-20}$ straight-chain alkylene group, especially ethylene, propylene, butylene, pentylene, hexylene, heptylene, octylene, nonylene, decylene, undecylene, or dodecylene; and

$P^2$ represents a polymerizable group such as : $CH_2=CW^3-$, $CH_2=CW^3-(CO)_{p3}O-$,

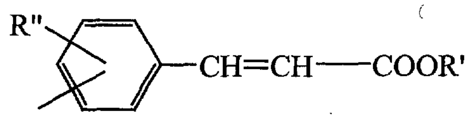

or

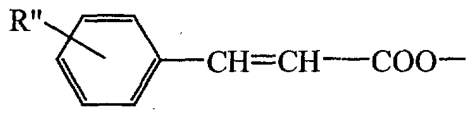

wherein:

$W^3$ represents H, $CH_3$, F, Cl, Br or I; and

p3 is 0 or 1.

More preferably, the group $P^2$ represents a polymerizable group $CH_2=CW^3-$ or $CH_2=CW^3-(CO)_{p3}O-$, wherein $W^3$ is H, $CH_3$, F, Cl, Br or I; and p3 is 0 or 1.

The group $Q^1$ is preferably selected from a residue of formula (IV):

$$P^3\text{-}Sp^1\text{-}(Y^1)_{k1}\text{-} \qquad \text{IV}$$

wherein:

$P^3$ is hydrogen or a polymerizable group $P^1$ with the meaning given above; and

$Sp^1$, $Y^1$ and k1 have the meaning given above.

More preferably, $Q^1$ is selected from a residue of formula (V):

$$P^4\text{-}Sp^2\text{-}Y^2\text{-} \qquad \text{V}$$

wherein:

$P^4$ is hydrogen or a polymerizable group $P^2$ with the meaning given above; and

$Sp^2$ and $Y^2$ have the meaning given above.

The group $Q^2$ is preferably selected from a residue of formula (VI):

$$P^5\text{-}(Sp^1)_{m1}\text{-}(Y^1)_{k1}\text{-} \qquad \text{VI}$$

wherein:

$P^5$ is hydrogen; halogen; a polar group such as -CN, and $-NO_2$; or a polymerizable group $P^1$ with the meaning given above and with the proviso that $Q^1$ does not comprise a polymerizable group; and

$Sp^1$, m1, $Y^1$ and $k^1$ have the meaning given above.

More preferably, $Q^2$ is selected from a residue of formula (VII):

$$P^5\text{-}(Sp^2\text{-}Y^2)_{m3}\text{-} \qquad \text{VII}$$

wherein:

| | |
|---|---|
| m3 | is an integer having a value of 0 orl; |
| $P^5$, $Sp^2$ and $Y^2$ | have the meaning given above. |

In a second preferred embodiment of the present invention, the groups $C^1$ and $C^3$ are selected from:

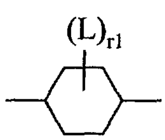 , 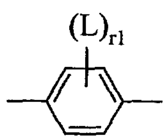 , 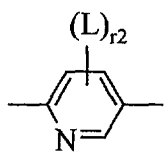 , 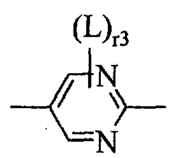 , 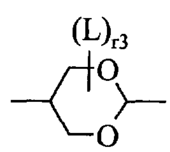 ,

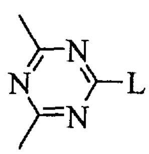 , 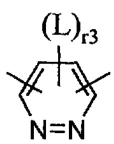 , 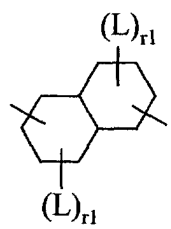 , 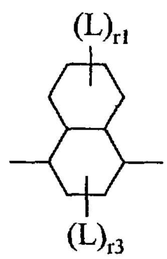 , 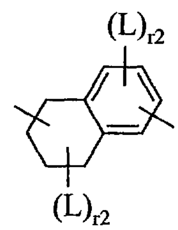 ,

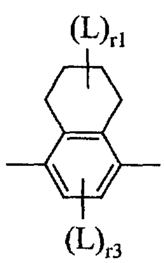 , 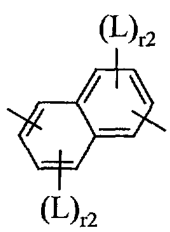 , 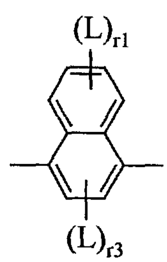 , 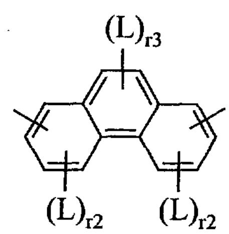 ,

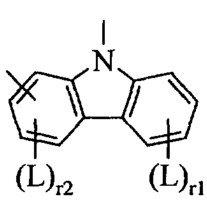 , 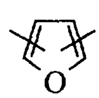 , 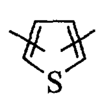 , 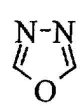 and 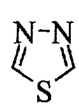

with:

| | |
|---|---|
| L | being $-CH_3$, $-COCH_3$, $-NO_2$, -CN, or halogen |
| r1 | being 0, 1, 2, 3, or 4, |
| r2 | being 0, 1, 2, or 3, and |

r3 being 0, 1, or 2.

It is especially preferred that $C^1$ and $C^3$ are selected from the group consisting of optionally-substituted trans-1,4-cyclohexylene, 1,4-phenylene and 2,6- naphthylenediyl.

The group $C^2$ is preferably selected from:

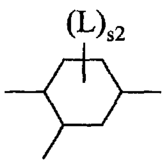 , 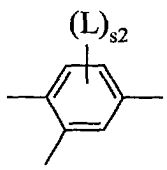 , 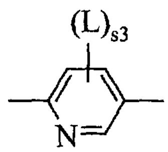 , 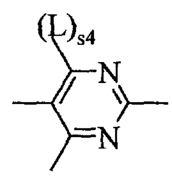 , 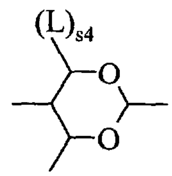 ,

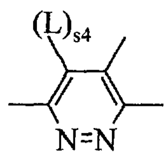 , 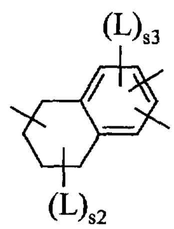 , 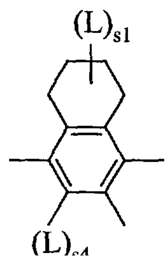 , 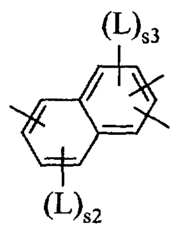 ,

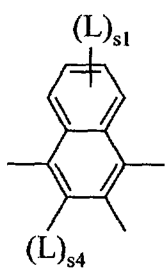 , 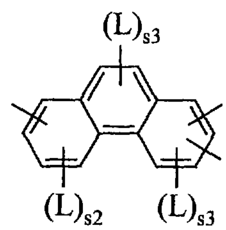 , 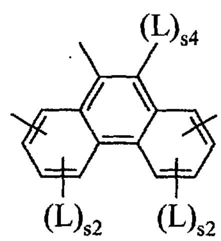 ,

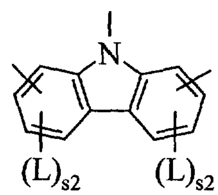 , 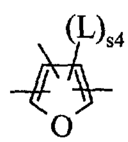 and 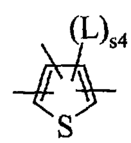

with:

- L being $-CH_3$, $-COCH_3$, $-NO_2$, -CN, or halogen,
- s1 being 0, 1, 2, 3, or 4,
- s2 being 0, 1, 2, or 3,
- s3 being 0, 1, or 2 and
- s4 being 0 or 1.

It is especially preferred that $C^2$ is selected from the group consisting of optionally-substituted 1,2,4-cyclohexatriyl, 1,2,4-benzenetriyl, 1,2,4-naphthenetriyl, 1,2,6-naphthenetriyl and 2,3,6-naphthenetriyl.

The groups $X^1$ and $X^2$ are preferably independently selected from the group consisting of -COO-, -OCO-, $-CH_2-CH_2-$, $-CH_2O-$, $-OCH_2-$, -CH=CH-, -C≡C- and a single bond.
It is especially prefered that $X^1$ and $X^2$ are independently selected from the group consisting of of -COO-, -OCO- and a single bond.
The integers n1 and n2 have preferably independently a value from 1 to 3. It is especially preferred that the sum of n1 + n2 is 2, 3 or 4.
A further preferred embodiment of the present invention are compounds according to formula (I), in which:

A is a residue selected from the formula (VIII)

$$Y^3-(Sp^2)_{n3}-P^2 \qquad \text{VIII}$$

wherein:

$Y^3$ represents -O-, -COO-, -OCO- or a single bond;
n3 is an integer having a value of 0 orl; and
$Sp^2$ and $P^2$ have the meaning given above;

$Q^1$ is a residue selected from the formula (IX)

$$P^4-Sp^2-Y^3 \qquad \text{IX}$$

wherein:

$P^4$, $Sp^2$ and $Y^3$ have the meaning given above;

$Q^2$ is halogen; CN; a polymerizable group $P^2$ with the meaning given above and with the proviso that $Q^1$ does not comprise a polymerizable group; or a residue selected from the formula (X)

$$CH_3-(Sp^2)_{n3}-Y^3 \qquad \text{IX}$$

wherein:

$Sp^2$, n3 and $Y^3$ have the meaning given above;

$C^1$ to $C^3$, $X^1$ and $X^2$ have the meaning given above; and

n1 and n2 are integers, wherein the sum of n1 + n2 is 2, 3 or 4.

Furthermore, it should be understood that generally one or more hydrogen atoms in the compounds of the present invention may be replaced by deuterium, in particular at saturated carbon atoms and especially in saturated cyclic moieties such as cyclohexane radicals.
The compounds of the invention may be readily prepared using methods that are well known to the person skilled in the art, such as those documented in Houben-Weyl, *Methoden der Organischen Chemie,* Thieme-Verlag, Stuttgart. The compounds may for example be made according to the reaction schemes 1-4 in which the following abbreviation are used:

DEAD is Diethyl azodicarboxylate
TPP is Triphenylphosphine
THF is Tetrahydrofurane
DMF is N, N-Dimethylformamide
$Et_3N$ is Triethylamine
DCM is Dichloromethane
DCC is N, N'-dicyclohexylcarbodiimide

DMAP is 4-Dimethylaminopyridine
n-BuLi is n-Butyllithium
TMEDA is N,N,N',N'-Tetramethylethylenediamine
DBU is 1,8-Diazabicyclo[5.4.0]undec-7-en
EE is Ethylacetate
p-TsOH is p-Toluenesulfonic acid

Scheme 1:

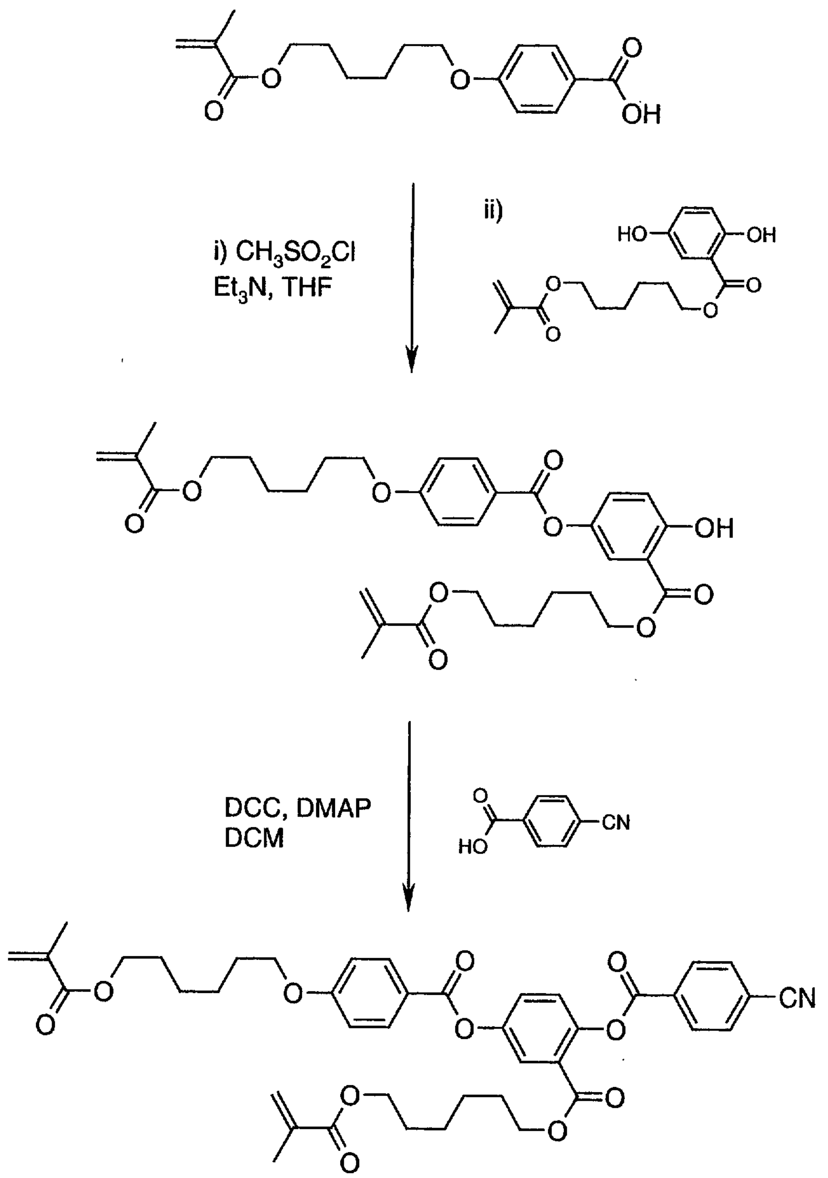

Scheme 2:

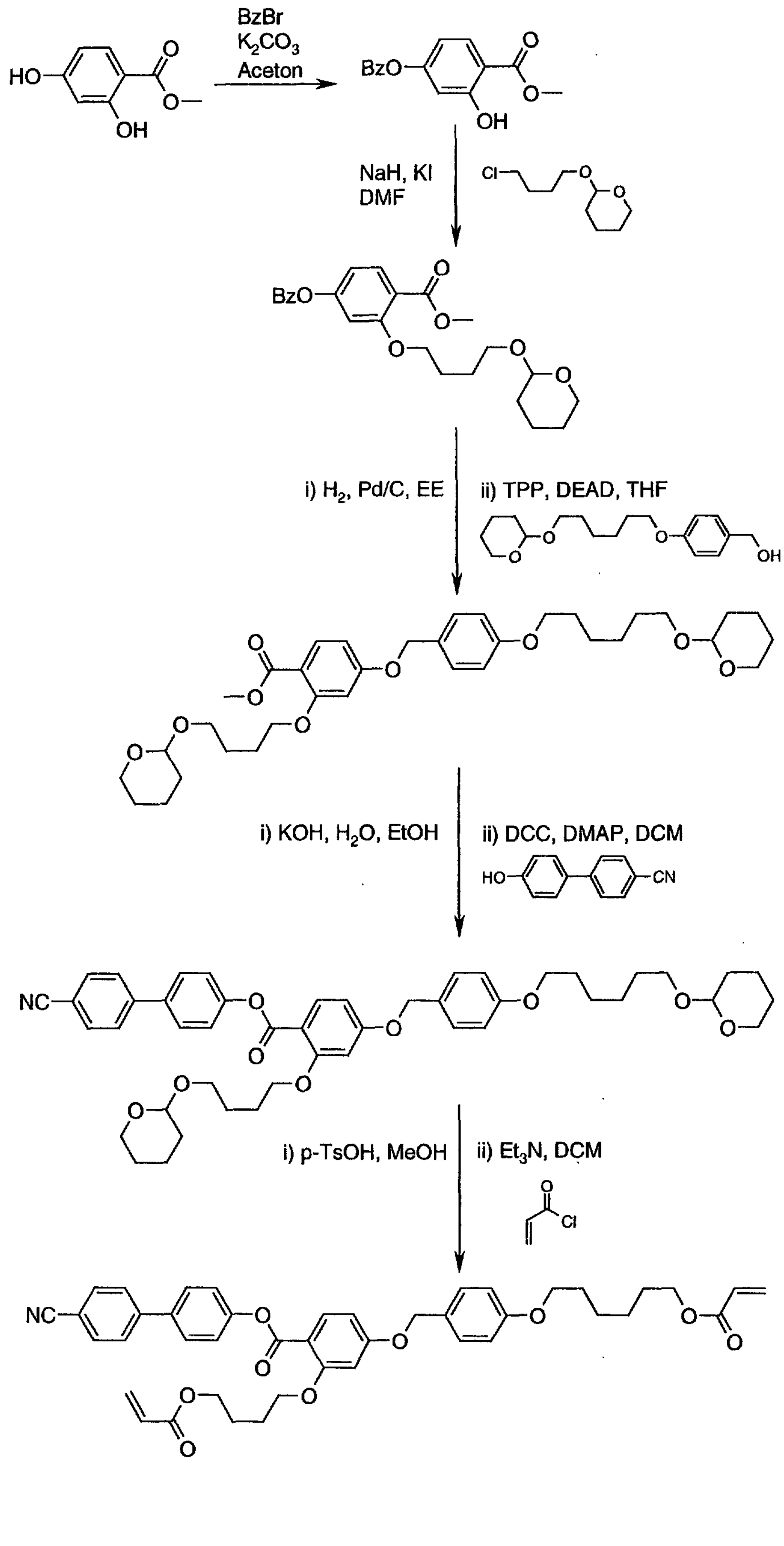
BzBr
$K_2CO_3$
Aceton
NaH, KI
DMF
i) $H_2$, Pd/C, EE
ii) TPP, DEAD, THF
i) KOH, $H_2O$, EtOH
ii) DCC, DMAP, DCM
i) p-TsOH, MeOH
ii) $Et_3N$, DCM

Scheme 3:

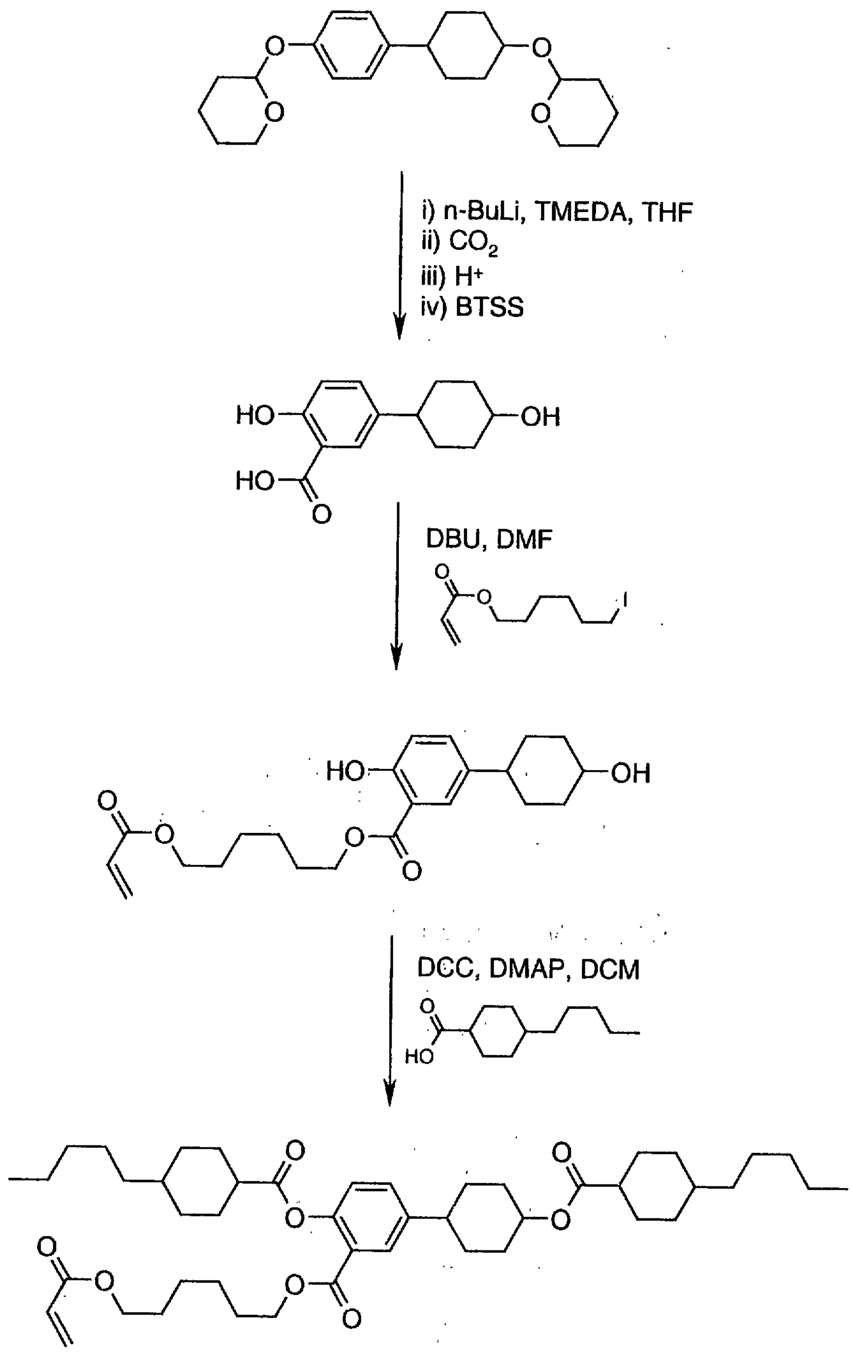

Scheme 4:

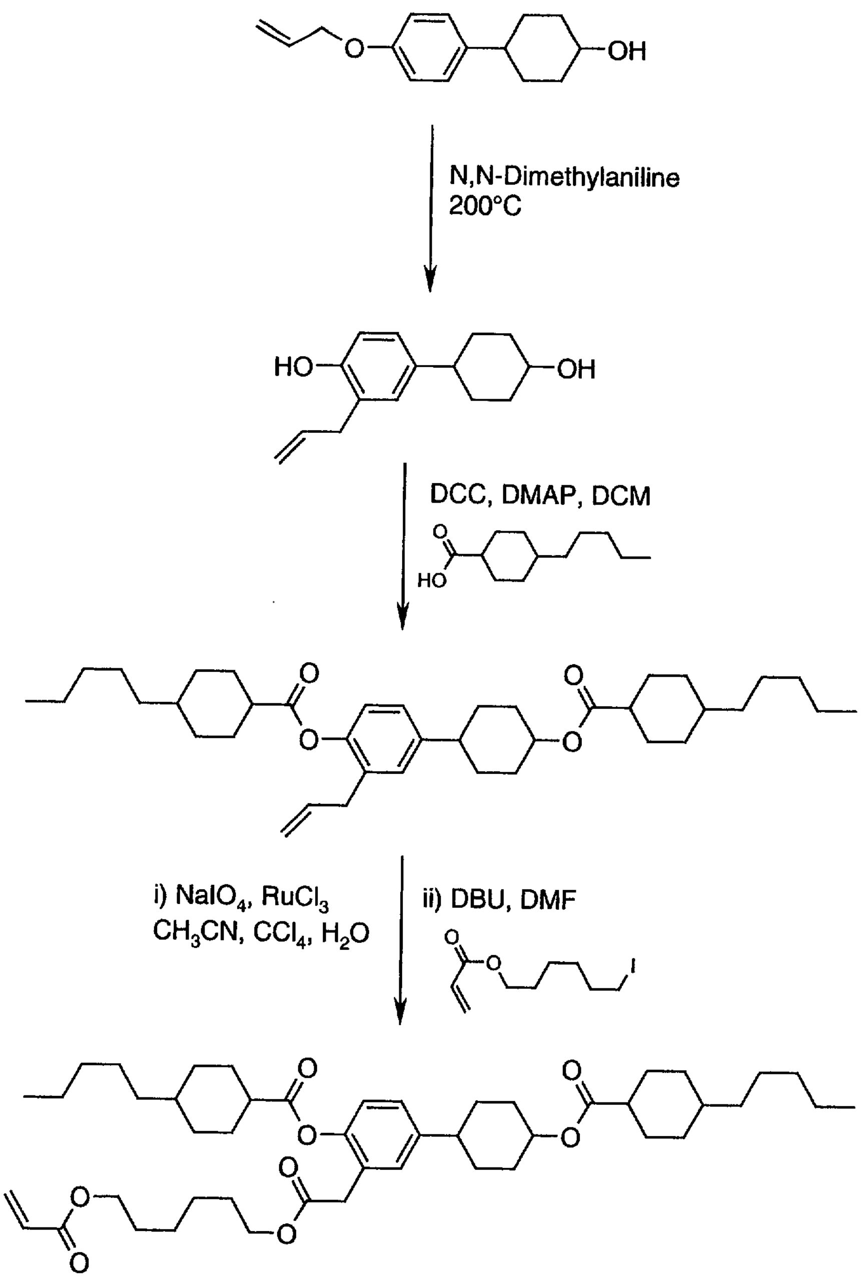


Based on the synthetic ways drawn in Schemes 1—4, typical examples representing "laterally polymerizable" derivatives of formula (I) and shown in the following list of compounds may be prepared. This list is, however, to be understood only as illustrative without limiting the scope of the present invention:

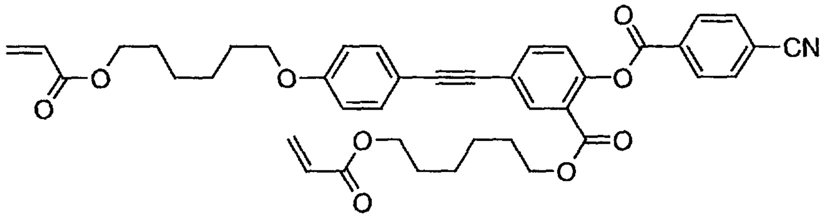

(I. 1)

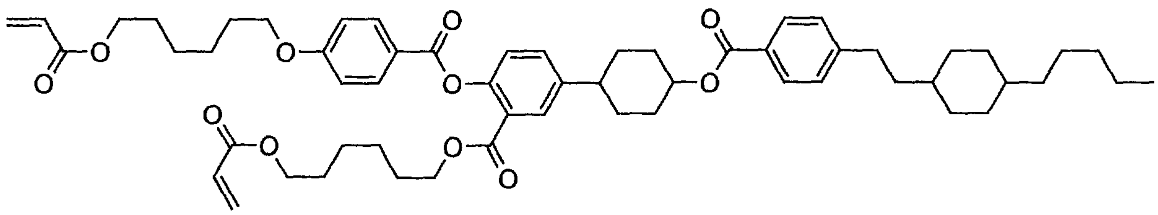

(I. 2)

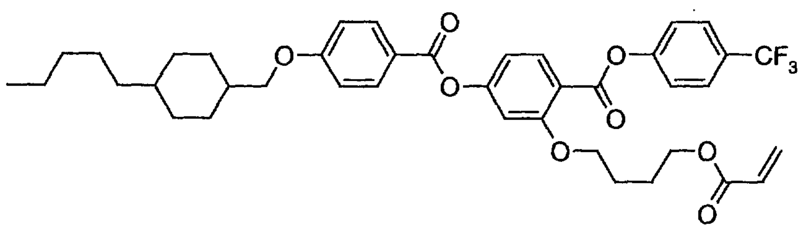

(I. 3)

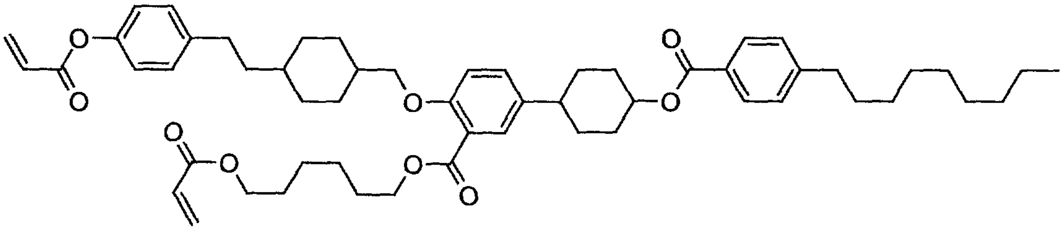

(I. 4)

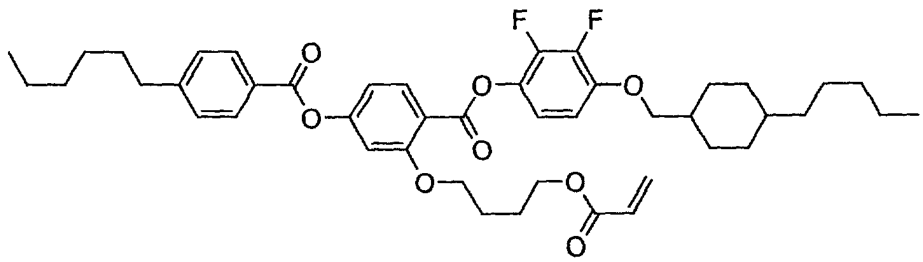

(I. 5)

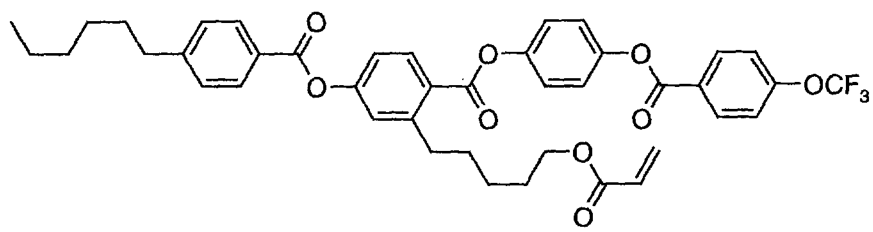

(I. 6)

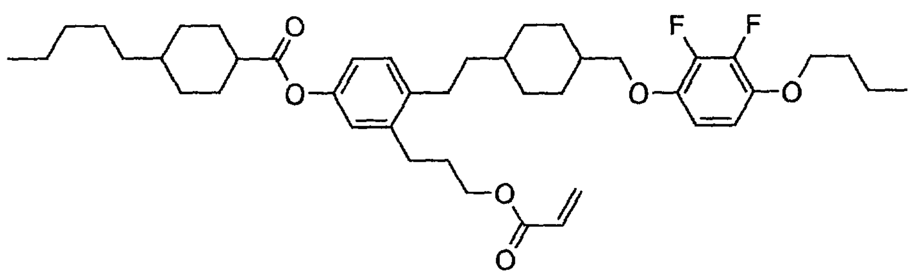

(I. 7)

The liquid crystalline "laterally polymerizable" compounds of formula (I) maybe used alone or as a component of a liquid crystal mixture. Liquid crystalline materials comprising a compound of formula (I) may be used in the manufacture of LCPs. A second aspect of the invention therefore comprises a liquid crystalline material comprising a compound of formula (I). Preferably, the liquid crystalline materials comprise at least two components. The additional components must be miscible with the compound of formula (I) and may be selected from known mesogenic materials such as those reported in *Adv. Mater.* 5, **107** (1993), *Mol. Cryst. Liq. Cryst.* **307,** 111 (1997), *J. Mat. Chem.* **5**, 2047 (1995) or in patents and patent applications US-A-5,593,617; US-A-5,567,349; GB-A-2 297 556; GB-A-2 299 333; US-A-5,560,864; EP-A-0 606 940; EP-A-0 643 121 and EP-A-0 606 939.

The form of the liquid crystal material will depend upon the application in which it is to be used and may be present as a liquid crystalline mixture, (co)polymer, elastomer, polymer gel or polymer network. Polymer networks have been found to be of particular use and in a further preferred embodiment of the invention there is provided a polymer network comprising a compound of formula (I). Preferably the polymer network comprises at least two components, at least one of which is a liquid crystalline "laterally polymerizable" compound of formula (I).

The polymer network may be prepared by copolymerization of a mesogenic mixture comprising:

i) at least one chiral or/and achiral mesogenic polymerizable compound;

ii) at least one " laterally polymerizable " compound of formula I; and
iii) an initiator.

The chiral or achiral mesogenic polymerizable compound may be a liquid crystalline "laterally polymerizable" compound of formula (I). Alternatively or in addition, the polymerizable compound may be selected from the known mesogenic materials referred to above. Preferably, the chiral or achiral polymerizable compound has a thermotropic sequence which includes a nematic phase.

The polymer network may optionally comprise further components. These include further polymerizable compounds, stabilizers and dyes. The additional polymerizable compounds preferably comprise a non-mesogenic compound having at least one polymerizable functional group, especially diacrylate compounds.
Any suitable stabilizer that prevents undesired spontaneous polymerization, for example during storage of the mixture, may be used in liquid crystalline mixtures of the present invention. A broad range of these compounds is commercially available. Typical examples include 4-ethoxyphenol or 2,6-di-tert-butyl-4-methylphenol (BHT).

For color filters, dyes may be added to the mixture. It is, however, preferred to prepare liquid crystalline mixtures containing no dye.

The chiral or achiral polymerizable mesogenic compound may be present in an amount comprising 0.01 to 99 % by weight of the polymer network, preferably 50 to 95 % by weight.

The liquid crystalline "laterally polymerizable" compound of formula (I) may be present in an amount from 0.1 to 100 % by weight of the polymer network, preferably from 1 to 50 % by weight.

The initiator is preferably a photoinitiator and may be a radical or cationic initiator that is present in an amount comprising 0.1 to 5 % by weight of the polymer network, preferably from 0.2 to 2 % by weight.

When the mixture further comprises stabilizers, these are generally present in an amount comprising 0.01 to 5 % by weight of the liquid crystalline mixture, preferably from 0.1 to 1 % by weight.

These polymerizable liquid crystalline mixtures may be formed into liquid crystalline polymer (LCP) networks in form of films and a third aspect of the invention provides a LCP film comprising a compound of formula (I). Such LCP networks in form of a film may be readily prepared by polymerization, e.g. UV polymerization, of a LC mixture according to the invention; a film comprising the LC mixture is formed on a substrate and polymerized using UV light to give a cross-linked liquid crystal polymer (LCP) film. The film is both light and temperature stable and can be used in the manufacture of devices such as waveguides, optical gratings, filters, retarders, rotators, piezoelectric cells or thin films exhibiting non-linear optical properties.

Different methods can be used for the formation of the LCP network. Transparent substrates such as coated ITO (indium tin oxide), glass, plastic or silicone substrates, may be used. Preferred substrates include glass or plastic, especially those including a layer of rubbed polyimide or polyamide or a layer of photo-oriented photopolymer such as a linearly photopolymerized polymer (LPP). The preferred substrates greatly facilitate uniform orientation of the liquid crystalline mixture.

In the preparation of LCP films, it is particularly important to prevent the formation of defects or inhomogenities. This can be achieved by forming the polymerizable liquid crystalline mixture into a thin film; and placing the mixture between two of the aforementioned substrates which are then sheared over a small distance until a planar order was obtained; or capillary filling the polymerizable liquid crystalline mixture between two of the said substrates; prior to curing, for example by UV light, preferably in the presence of a photoinitiator, such as IRGACURE™.

A fourth aspect of the invention provides an optical or electro-optical component containing a liquid crystalline polymer film comprising a compound of formula (I). The optical or electro-optical component may be a waveguide, an optical grating, a filter, a retarder, a rotator, a piezoelectric cell or a non-linear optical cell or film.

The invention will now be described with reference to the following examples. Variations on these falling within the scope of the invention will be apparent to a person skilled in the art.

In the following examples the thermotropic phases are abbreviated as follows: K for crystalline, S for smectic, N for nematic, and I for isotropic.

EXAMPLE 1:

6-(Methacryloyloxy)hexyl 2-[(4-cyanobenzoyl)oxy]-5-[(4-{[6-(methacryloyloxy) hexyl]oxy}benzoyl)oxy]benzoate:

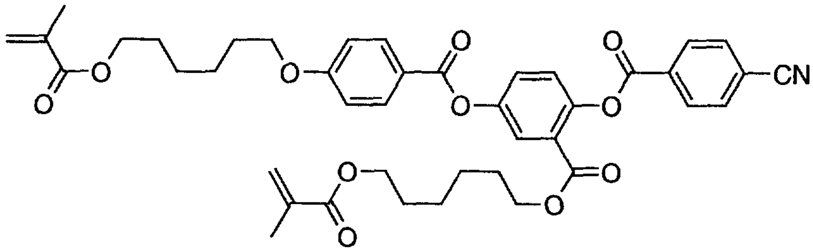

1) 6-(Methacryloyloxy)hexyl 2-hydroxy-5-[(4-{[6-(methacryloyloxy)hexyl]oxy} benzoyl)oxy]benzoate

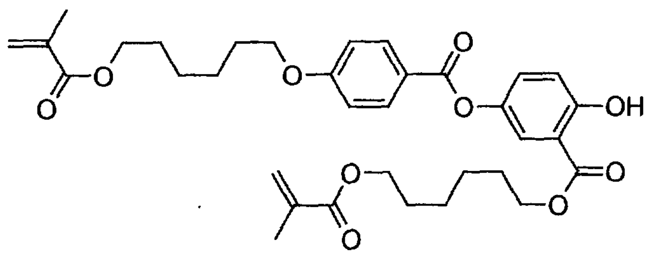

To a stirred solution of 4-{[6-(methacryloyloxy)hexyl]oxy}benzoic acid (7.82 g) in 60 ml of dry THF was added dropwise triethylamine (8.90 g) at —30 °C, followed by mesyl chlorid (2.92 g). After complete addition, the reaction mixture was further stirred for lh at —30 °C, then a solution of 6-(methacryloyloxy)hexyl 2,5-dihydroxybenzoate (7.09 g) in 30 ml of dry THF was added dropwise. The reaction mixture was further stirred at —30 °C for 30 min, then at room temperature overnight. The reaction mixture was filtered over celite and the filter cake was washed with ethyl acetate. The filtrate was poured on 150 ml of water, the organic phase separated and washed with 100 ml of water, dried over magnesium sulfate and evaporated to dryness. The residue was flash chromatographed on silica gel using cyclohexane / ethyl acetate : 3 / 1 as eluent to afford pure 6-(methacryloyloxy)hexyl 2-hydroxy-5-[(4-{[6-(methacryloyloxy)-hexyl]oxy}benzoyl)oxy]benzoate as colorless oil. Yield: 9.81 g.

2) 6-(Methacryloyloxy)hexyl 2-[(4-cyanobenzoyl)oxy]-5-[(4-{[6-(methacryloyloxy)hexyl]oxy}benzoyl)oxy]benzoate

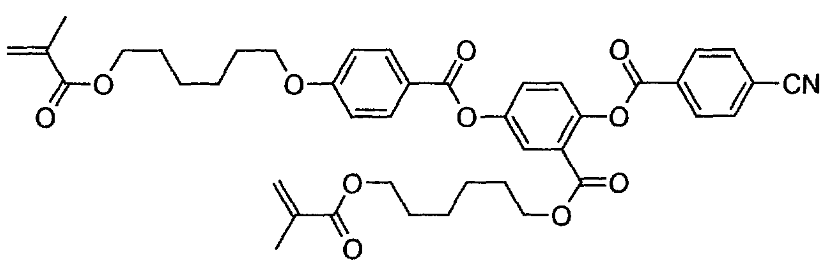

A solution of 6-(methacryloyloxy)hexyl 2-hydroxy-5-[(4-{[6-(methacryloyloxy)-hexyl]oxy}benzoyl)oxy]benzoate (1.83 g), 4-cyanobenzoic acid (0.48 g), DCC (0.68 g) and DMAP (0.41 g) in 20 ml of DCM was stirred overnight, filtered and the filtrate was evaporated to dryness. The residue was flash chromatographed on silica gel using cyclohexane / ethyl acetate : 11 / 4 as eluent to afford the desired product. Further purification by recrystallization from aceton / ethanol gave pure 6-(methacryloyloxy)-hexyl 2-[(4-cyanobenzoyl)oxy]-5-[(4-{[6(methacryloyloxy)

hexyl]oxy}benzoyl)oxy]-benzoate as white powder.
Yield 1.40 g.
This compound has the following thermotropic sequence:
Cr 54 °C (N 44 °C) I.

EXAMPLE 2:

6-(Methacryloyloxy)hexyl 2-({4-[(4-cyanobenzoyl)oxy]benzoyl}oxy)-5-[(4-{[6-(methacryloyloxy)hexyl]oxy}benzoyl)oxy] benzoate:

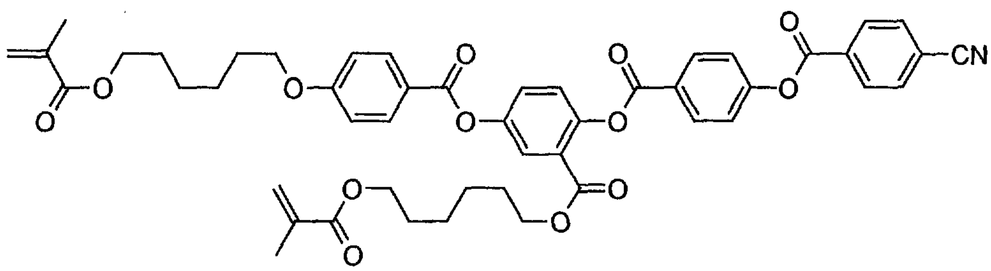

1) 6-(Methacryloyloxy)hexyl 5-[(4-{[6-(methacryloyloxy)hexyl]oxy}benzoyl)-oxy]-2-{[4-(tetrahydro-2*H*-pyran-2-yloxy)benzoyl]oxy}benzoate

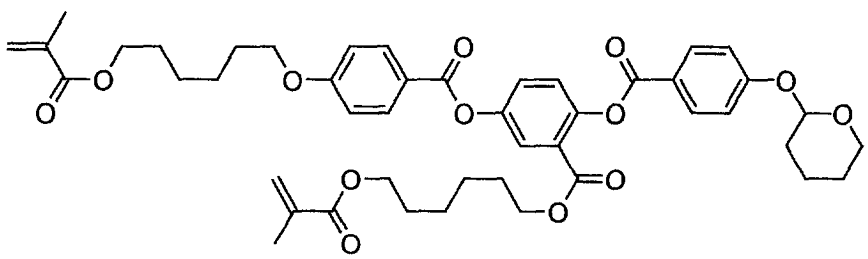

A solution of 6-(methacryloyloxy)hexyl 2-hydroxy-5-[(4- {[6-(methacryloyloxy)-hexyl]oxy}benzoyl)oxy]benzoate (2.44 g), 4-(tetrahydro-2*H*-pyran-2-yloxy)benzoic acid (0.98 g), DCC (0.90 g) and DMAP (0.54 g) in 20 ml of DCM was stirred overnight, filtered and the filtrate was evaporated to dryness. The residue was flash chromatographed on silica gel using cyclohexane / ethyl acetate : 11 / 4 as eluent to afford pure 6-(methacryloyloxy)hexyl 5-[(4-{[6-(methacryloyloxy)hexyl]-oxy}benzoyl)oxy]-2-{[4-(tetrahydro-2*H*-pyran-2-yloxy)benzoyl]oxy}benzoate as colorless oil.
Yield 3.25 g.

2) 6-(Methacryloyloxy)hexyl 2-[(4-hydroxybenzoyl)oxy]-5-[(4-{[6-(methacryloyloxy)hexyl]oxy}benzoyl)oxy]benzoate

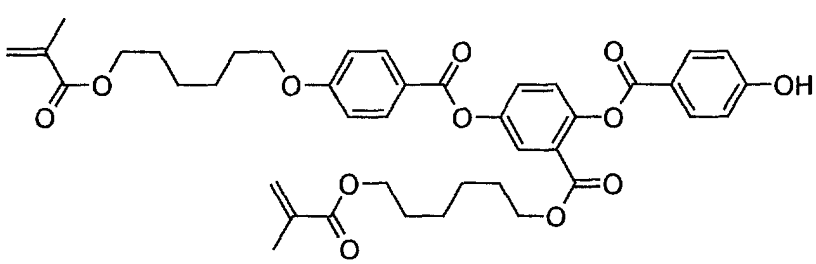

A stirred solution of 6-(methacryloyloxy)hexyl 5-[{4-{[6-(methacryloyloxy)hexyl]-oxy}benzoyl)oxy]-2-{[4-(tetrahydro-2*H*-pyran-2-yloxy)benzoyl]oxy}benzoate (3.10 g) and BTSS (0.05 g) in 30 ml of methanol was refluxed for 3 h. The reaction mixture was then evaporated, poured on 50 ml of ethyl acetate, washed with 50 ml of water, dried over sodium sulfate and evaporated to dryness. The residue was flash chromatographed on silica gel using cyclohexane / ethyl acetate : 3 / 1 as eluent to afford pure 6-(methacryloyloxy)hexyl 2-[(4-hydroxybenzoyl)oxy]-5-[(4-{[6-(methacryloyl-oxy)-hexyl]oxy}benzoyl)oxy]benzoate as colourless oil.
Yield 1.22 g.

3) 6-(Methacryloyloxy)hexyl2-({4-[(4-cyanobenzoyl)oxy]benzoyl}oxy)-5-[(4-{[6-(methacryloyloxy)hexyl]oxy}benzoyl)oxy]benzoate

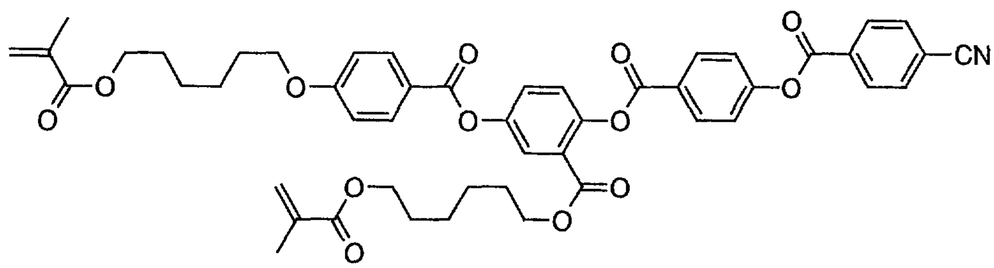

A solution of 6-(methacryloyloxy)hexyl 2-[(4-hydroxybenzoyl)oxy]-5-[(4-{[6-(methacryloyl-oxy)-hexyl]oxy}benzoyl)oxy]benzoate (1.10 g), 4-cyanobenzoic acid (0.23 g), DCC (0.37 g) and DMAP (0.22 g) in 30 ml of DCM was stirred overnight, filtered and the filtrate was evaporated to dryness. The residue was flash chromatographed on silica gel using cyclohexane / ethyl acetate : 3 / 1 as eluent to afford the desired product. Further purification by recrystallization from aceton / ethanol gave pure 6-(methacryloyloxy)hexyl 2-({4-[(4-cyanobenzoyl)oxy]benzoyl}oxy)-5-[(4- {[6-(methacryloyloxy)hexyl]oxy}benzoyl)oxy]benzoate as white powder.
Yield 0.83 g.
This compound has the following thermotropic sequence:
Cr 89 °C N 160 °C I .

<u>EXAMPLE 3:</u>

<u>6-(Acryloyloxy)hexyl 2-{[(4-pentylcyclohexyl)carbonyl]oxy}-5-(4-{[(4-pentylcyclohexyl)carbonyl]oxy}cyclohexyl)benzoate;</u>

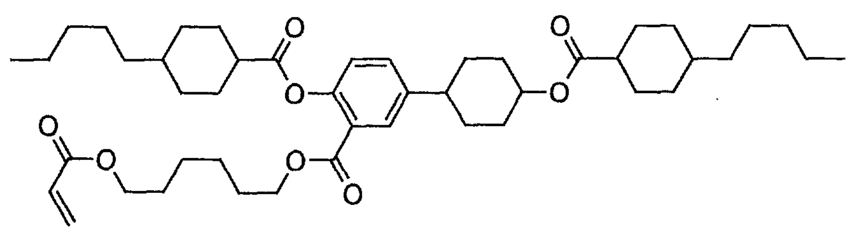

1) 2-Hydroxy-5-(4-hydroxycyclohexyl)benzoic acid

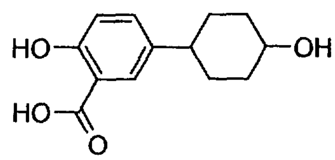

To a stirred solution of 2-{4-[4-(tetrahydro-2*H*-pyran-2-yloxy)cyclohexyl]phenoxy}tetrahydro-2*H*-pyran (16.60 g) in 100 ml of dry THF at 0 °C under an atmosphere of argon, was added TMEDA (6.41 g), followed by n-BuLi (20.4 ml of a 2.7 M solution in n-heptane). The resulting mixture was stirred for 1h at room temperature, chilled to -78 °C and solid $CO_2$ (approx. 5 g) was added. The mixture was allowed to warm to room temperature and then quenched with 100 ml of a 5N HCl. The resulting mixture was poured on 250 ml ethyl acetate and the organic phase was separated, washed with 50 ml of water, dried over magnesium sulfate and evaporated to dryness. To the residue was added 300 ml of methanol and BTSS (0.50 g) and the resulting solution was refluxed for 1h, then evaporated to yield a beige solid, which was recrystallized from ethyl acetat / hexanes to afford pure 2-hydroxy-5-(4-hydroxycyclohexyl)benzoic acid as white powder.
Yield 6.92 g.

2) 6-(Acryloyloxy)hexyl 2-hydroxy-5-(4-hydroxycyclohexyl)benzoate

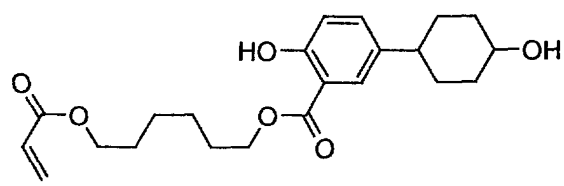

To a stirred solution of 2-hydroxy-5-(4-hydroxycyclohexyl)benzoic acid (5.90 g) in 30 ml of DMF was added DBU (3.81 g), followed by a solution of 6-iodohexyl acrylate (8.50 g) in 10 ml of DMF. The mixture was stirred for 4h at 45 °C and then at room temperature overnight. The reaction mixture was poured on 150 ml of ethyl acetate and washed with 100 ml of a 2N HCl. The aqueous phase was extracted with 50 ml of ethyl acetate and the combined organic phases were washed with half saturated $NaHCO_3$ solution (150 ml), then with half saturated NaCl solution (150 ml), dried over sodium sulfate and evaporated to dryness. The residue was flash chromatographed on silica gel using cyclohexane / ethyl acetate : 2 / 1 as eluent to afford pure 6-(acryloyloxy)hexyl 2-hydroxy-5-(4-hydroxycyclohexyl)benzoate as colorless oil.
Yield 5.00 g.

3) 6-(Acryloyloxy)hexyl 2-{[(4-pentylcyclohexyl)carbonyl]oxy}-5-(4- {[(4-pentylcyclohexyl)carbonyl]oxy} cyclohexyl)benzoate

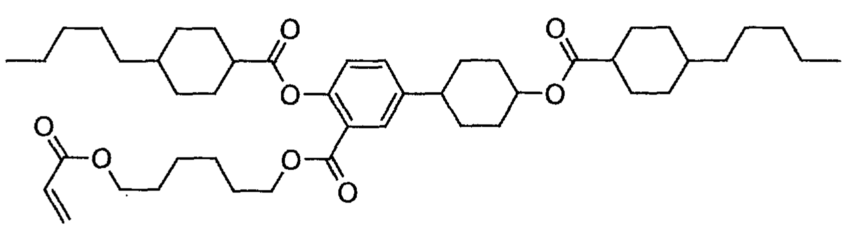

A solution of 6-(acryloyloxy)hexyl 2-hydroxy-5-(4-hydroxycyclohexyl)benzoate (2.40 g), 4-pentylcyclohexanecarboxylic acid (3.80 g), DCC (3.90 g) and DMAP (2.30 g) in 30 ml of DCM was stirred overnight, filtered and the filtrate evaporated to dryness. The residue was flash chromatographed on silica gel using cyclohexane / ethyl acetate : 15 / 1 as eluent to afford the desired product. Further purification by recrystallization from aceton / ethanol gave pure 6-(acryloyloxy)hexyl 2-{[(4-pentylcyclohexyl)-carbonyl]oxy}-5-(4-{[(4-pentylcyclohexyl)carbonyl]oxy}cyclohexyl)benzoate as white powder.

Yield 2.86 g.
This compound has the following thermotropic sequence:
Cr 64 °C N 101 °C I.

The following compounds were prepared in a similar way.

6-(Acryloyloxy)butyl 2-{[(4-propylcyclohexyl)carbonyl]oxy}-5-(4-{[(4-propylcyclohexyl)carbonyl]oxy}cyclohexyl) benzoate

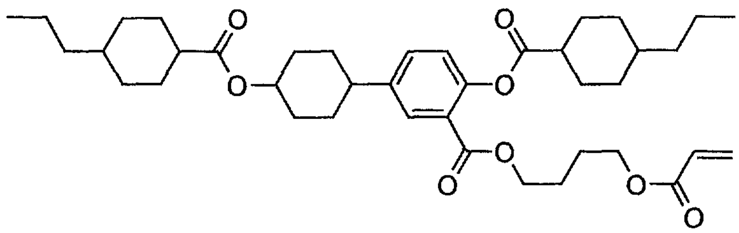

Cr 48 °C N 101 °C I .

6-(Acryloyloxy)butyl 2-{[(4-pentylcyclohexyl)carbonyl]oxy}-5-(4-{[(4-pentylcyclohexyl)carbonyl]oxy}cyclohexyl) benzoate

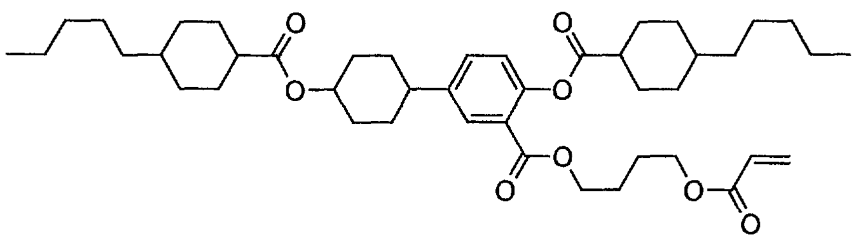

Cr 71 °C N 123 °C I.

6-(Acryloyloxy)butyl 2-{[(4-heptylcyclohexyl)carbonyl]oxy}-5-(4-{[(4-heptylcyclohexyl)carbonyl]oxy}cyclohexyl) benzoate

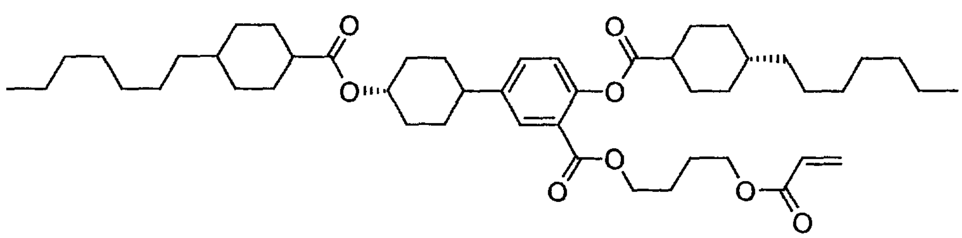

Cr 74 °C S 97 °C N 136 °C I.

2-({[6-(Acryloyloxy)hexyl]oxy}carbonyl)-4-{[(4-pentylcyclohexyl)carbonyl]oxy}phenyl 4'-(4-pentylcyclohexyl)-1,1'-biphenyl-4-carboxylate:

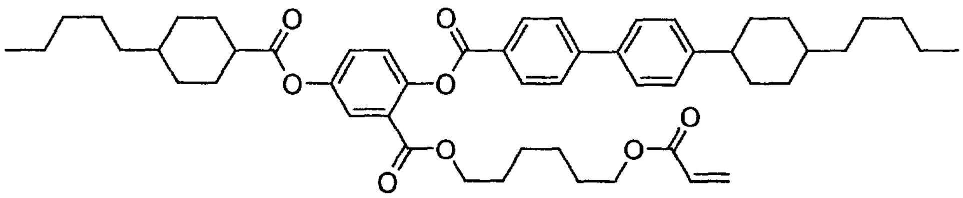

Cr 65 °C N >150 °C I .

EXAMPLE 4:

2-(2-{[6-(Acryloyloxy)hexyl]oxy}-2-oxoethyl)-4-(4-{[(4-pentylcyclohexyl)carbonyl]-oxy}cyclohexyl) phenyl4-pentylcyclohexanecarboxylate

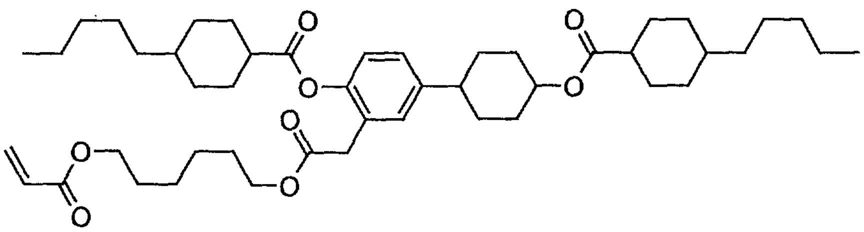

1) 2-Allyl-4-(4-hydroxycyclohexyl)phenol

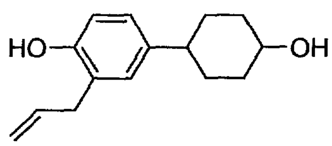

A solution of 4-[4-(allyloxy)phenyl]cyclohexanol (22.90 g) in 100 ml of N,N-dimethylaniline was stirred at 200 °C under an atmosphere of argon for 24 h. The mixture was cooled to room temperature and then poured on 500 ml ethyl acetate washed 4 times each with 250 ml of a 5N HCl. The organic phase was dried over magnesium sulfate and evaporated to dryness to afford crude 2-Allyl-4-(4-hydroxycyclohexyl)phenol as grey powder, which was used without further purification. Yield 22.43 g.

2) 2-Allyl-4-(4- {[(4-pentylcyclohexyl)carbonyl]oxy} cyclohexyl)phenyl 4-pentylcyclohexanecarboxylate

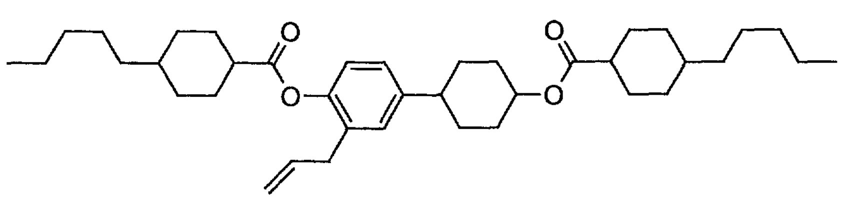

A solution of crude 2-Allyl-4-(4-hydroxycyclohexyl)phenol (8.13 g), 4-pentylcyclohexanecarboxylic acid (17.35 g), DCC (15.90 g) and DMAP (9.40 g) in 400 ml of DCM was stirred overnight, filtered and the filtrate evaporated to dryness. The residue was flash chromatographed on silica gel using cyclohexane / ethyl acetate : 9 / 1 as eluent to afford pure 2-allyl-4-(4-{[(4-pentylcyclohexyl)carbonyl]oxy}cyclohexyl)phenyl 4-pentyl-cyclohexanecarboxylate as light brown powder. Yield 20.75 g.

3) 2-(2-{[6-(Acryloyloxy)hexyl]oxy}-2-oxoethyl)-4-(4-{[(4-pentylcyclohexyl)-carbonyl]oxy}cyclohexyl)phenyl 4-pentylcyclohexanecarboxylate

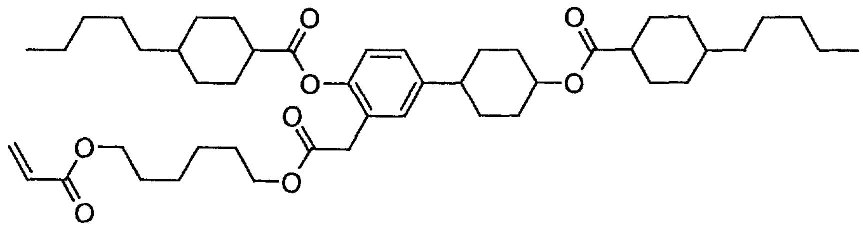

2-Allyl-4-(4- {[(4-pentylcyclohexyl)carbonyl]oxy} cyclohexyl)phenyl 4-pentylcyclohexanecarboxylate (20.75 g) was suspended in 120 ml of Acetonitrile, 120 ml of tetrachloromethane and 240 ml of water. To this suspension were added $NaIO_4$ (74.90 g) and $RuCl_3$ (0.27 g) and the reaction mixture was stirred overnight and then poured on 250 ml of DCM and washed with 200 ml of water. The aqueous phase was extracted with 50 ml of DCM and the combined organic phases were washed with 200 ml of water, dried over sodium sulfate and evaporated to dryness to yield 28.90 g of a grey powder of crude [2-{[(4-pentylcyclohexyl)carbonyl]oxy}-5-(4-{[(4-pentylcyclohexyl)carbonyl]oxy}cyclohexyl)phenyl]acetic acid, which was used in the next step without further purification.
To a stirred solution of crude [2-{[(4-pentylcyclohexyl)carbonyl]oxy}-5-(4-{[(4-pentylcyclohexyl)carbonyl]oxy}cyclohexyl)phenyl] acetic acid as prepared according to above (9.60 g) in 100 ml of DMF was added DBU (1.75 g), followed by a solution of 6-iodohexyl acrylate (3.23 g) in 50 ml of DMF. The mixture was stirred for 3 h at 45 °C and then at room temperature overnight. The reaction mixture was poured on 300 ml of ethyl acetate and washed with 200 ml of a 2N HCl. The aqueous phase was extracted with 50 ml of ethyl acetate and the combined organic phases were washed with half saturated $NaHCO_3$ solution (250 ml), then with half saturated NaCl solution (250 ml), dried over magnesium sulfate and evaporated to dryness. The residue was flash chromatographed on silica gel using cyclohexane / ethyl acetate : 9 / 1 as eluent to afford the desired product. Further purification by recrystallization from methyl-tbutylether gave pure 2-(2-{[6-(Acryloyloxy)hexyl]oxy}-2-oxoethyl)-4-(4-{[(4-pentylcyclohexyl)-carbonyl]-oxy}cyclohexyl)phenyl 4-pentylcyclohexane-carboxylate as white powder.
Yield 1.16 g.
This compound has the following thermotropic sequence:
Cr 70 °C N 109 °C I .

The following compounds were prepared in a similar way.

<u>2-(2-{[6-(Acryloxyloxy)butyl]oxy}-2-oxoethyl)-4-(4-{[(4-pentylcyclohexyl)-carbonyl]-oxy}cyclohexyl)phenyl 4-pentylcyclohexanecarboxylate</u>

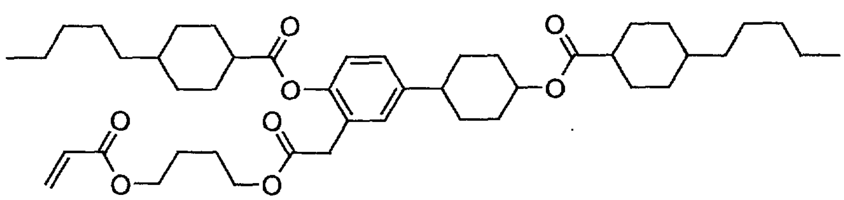

Cr 49 °C S 61 °C N 119 °C I.

EXAMPLE 5:

2-(Vinyloxy)ethyl 2,5-bis[(4-butoxybenzoyl)oxy]benzoate:

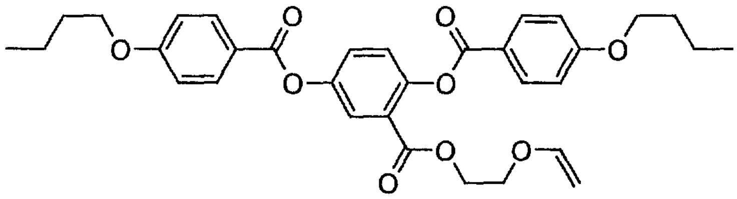

To a stirred solution of 2,5 dihydroxy benzoic acid (10.02 g) and ethylene glycol vinylether (5.45 g) in 250 ml of dry THF was added triphenylphosphine (17.0 g) at 5 °C, followed by a solution of diisopropyl azodicarboxylate (13.8 g) in 10 ml of dry THF. After complete addition, the reaction mixture was further stirred for 5 h at room temperature, then poured on 150 ml of water and extracted with 300 ml of ethyl acetate. The organic phase was separated and washed with 100 ml of water, dried over magnesium sulfate and evaporated to dryness. The residue was flash chromatographed on silica gel using cyclohexane / ethyl acetate / $Et_3N$: 3 / 1 / 0.005 as eluent to afford 30 g of crude 2-(vinyloxy)ethyl 2,5-dihydroxybenzoate as bright yellow oil, containing 1,2-dicarbisopropoxyhydrazine as by-product. 15 g of this mixture and 4-butoxy benzoic acid (14.55 g) were suspended in 200 ml of DCM and DCC (13.6 g) and DMAP (8.06 g) were added at 5 °C. The mixture was then stirred overnight at room temperature, filtered and the filtrate evaporated to dryness. The residue was flash chromatographed on silica gel using cyclohexane / ethyl acetate : 9 / 1 as eluent to afford pure 2-(vinyloxy)ethyl 2,5-bis[(4-butoxybenzoyl)oxy]benzoate as white powder. Yield 13.28 g.
This compound has the following thermotropic sequence:
Cr 88 °C N 119 °C I.

EXAMPLE 6:

2-{4-[(1*E*)-3-Methoxy-3-oxoprop-1-enyl]phenoxy}ethyl 2,5-bis[(4-butoxybenzoyl)oxy] benzoate:

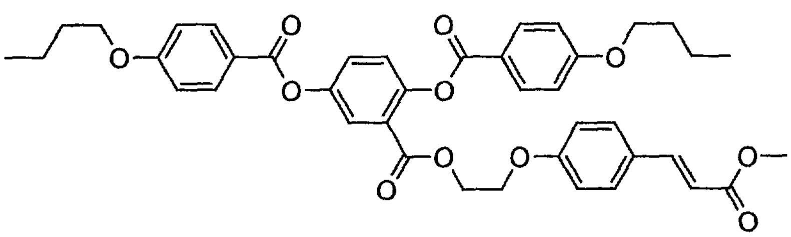

1) 2-Hydroxyethyl 2,5-bis[(4-butoxybenzoyl)oxy]benzoate

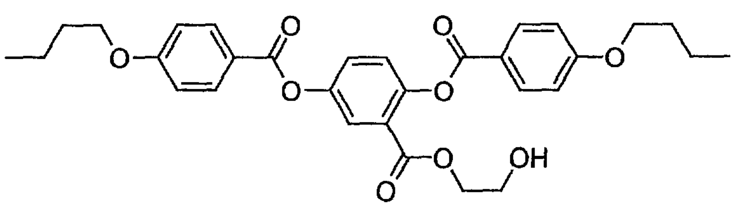

A solution of 2-(vinyloxy)ethyl 2,5-bis[(4-butoxybenzoyl)oxy]benzoate (12.50 g) in 300 ml of acetone, 50 ml of water

and 10 ml of hydrochloric acid (25 %) was stirred overnight. The acetone was evaporated and the residue was poured on 150 ml of water and extracted with 150 ml of DCM. The organic phase was separated and washed with 50 ml of water, dried over magnesium sulfate and evaporated to dryness to afford pure 2-hydroxyethyl 2,5-bis[(4-butoxybenzoyl)oxy]benzoate as white powder.
Yield 11.77 g.

2) 2-{4-[(1*E*)-3-Methoxy-3-oxoprop-1-enyl]phenoxy}ethyl 2,5-bis[(4-butoxybenzoyl)oxy] benzoate

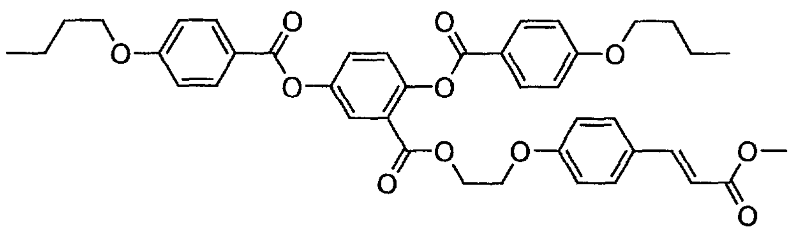

To a stirred solution of 2-hydroxyethyl 2,5-bis[(4-butoxybenzoyl)oxy]benzoate (1.75 g) and methyl (2*E*)-3-(4-hydroxyphenyl)prop-2-enoate (0.54 g) in 30 ml of dry THF was added triphenylphosphine (0.83 g) at 5 °C, followed by a solution of diisopropyl azodicarboxylate (0.67 g) in 5 ml of dry THF. After complete addition, the reaction mixture was further stirred overnight at room temperature, then evaporated to dryness. The residue was flash chromatographed on silica gel using DCM / ethyl acetate: 20 / 1 as eluent to afford 1.23 g of the desired product. Further purification by recrystallization from ethyl acetate / hexanes gave pure 2-{4-[(1*E*)-3-methoxy-3-oxoprop-1-enyl]phenoxy}ethyl 2,5-bis[(4-butoxybenzoyl)oxy] benzoate as white powder.
Yield 1.22 g.
This compound has the following thermotropic sequence:
C 139 °C N 173 °C I.

EXAMPLE 7:

Preparation of Nematic LCP Films

A solution of the following component in Anisole was prepared: 97 wt% of

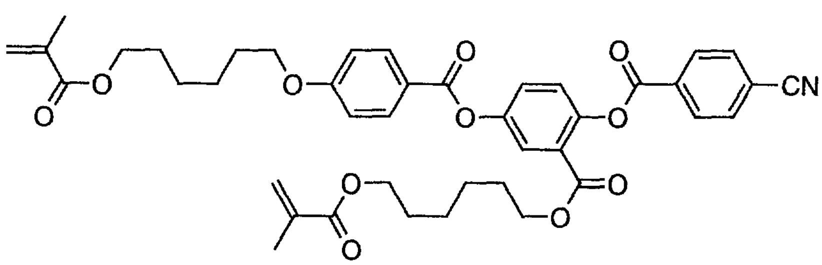

Further 1000 ppm Tinuvin 123 were added as a stabilizer, 1000 ppm of 2,6-di-(t-butyl)-4-hydroxytoluene (BHT) inhibitor were added to this mixture in order to prevent polymerization. Polymerization was started using 1000 ppm initiator such as Irgacure 369 ( commercially available from Ciba Geigy, Basel, Switzerland ). The mixture was stirred at room temperature and than spincoated on a glass plate having an orientation layer to form an LCP film of ca. 800nm in thickness. This film was dried at 50 °C for 1 or 2 minutes and photopolymerized by irradiation with UV light for approximately 5 minutes at room temperature in a $N_2$ atmosphere using a mecury lamp. The well oriented film shows the nematic mesophase at room temperature. In addition, this film exhibits a mean tilt angle of about 50° relative to the plane of the substrate, as shown by ellipsometric measurements.

## Claims

**1.** Compounds of formula (I):

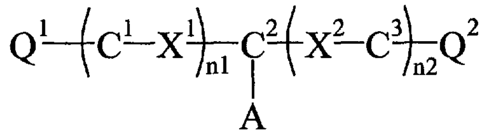

I

wherein:
$-(C^1-X^1)_{n1}-C^2-(X^2-C^3)_{n2}-$ is a rod-like core formed by a substantially linear arrangement of the residues represented by the symbols;

| | |
|---|---|
| A | includes a polymerizable group and represents an optionally-substituted methyl group; or an optionally-substituted hydrocarbon group of 2 to 20 C-atoms, in which one or more C-atoms may be replaced by a heteroatom, in such a way that oxygen atoms are not linked to one another; |
| $C^1$ to $C^3$ | are in each case independently optionally-substituted non-aromatic, aromatic, carbocyclic or heterocyclic groups; preferably connected to each other at the opposite positions via the bridging groups $X^1$ and $X^2$; |
| $Q^1$ | represents an optionally-substituted methyl group; or an optionally-substituted hydrocarbon group of 2 to 20 C-atoms, in which one or more C-atoms may be replaced by a heteroatom, in such a way that oxygen atoms are not linked to one another; and which may comprise a polymerizable group; |
| $Q^2$ | represents hydrogen; halogen; a polar group such as -CN and $-NO_2$; an optionally-substituted methyl group; an optionally-substituted hydrocarbon group of 2 to 20 C-atoms, in which one or more C-atoms may be replaced by a heteroatom, in such a way that oxygen atoms are not linked to one another; and may comprise a polymerizable group when $Q^1$ does not comprise a polymerizable group; |
| $X^1$ and $X^2$ | and each independently represent -O-, -S-, -NH-, -N(CH3)-, -N=N-, -CO-C=C-, -CH(OH)-, -CO-, $-CH_2(CO)-$, -SO-, $-CH_2(SO)-$, $-SO_2-$, $-CH_2(SO_2)-$, -COO-, -OCO-, -OCO-O-, -S-CO-, -CO-S-, -SOO-, -OSO-, -SOS-, $-CH_2-CH_2-$, $-OCH_2-$, $-CH_2O-$, -CH=CH-, -C≡C- or a single bond; and |
| n1 and n2 | are integers, each independently having a value from 1 to 4. |

**2.** Compound according to claim 1, wherein hydrocarbon includes straight-chain and branched alkylene, as well as saturated and unsaturated groups.

**3.** Compound according to claims 1 or 2, wherein the substituents include alkyl, aryl, cycloalkyl, amino, cyano, epoxy, halogen, hydroxy, nitro, and oxo groups.

**4.** Compound according to anyone of claims 1 to 3, wherein the heteroatoms which may replace carbon atoms include nitrogen, oxygen and sulphur.

**5.** Compound according to claim 4, wherein the nitrogen atom is substituted with groups such as alkyl, aryl and cycloalkyl.

**6.** Compound according to anyone of claims 1 to 5, wherein the group A may be selected from a residue of formula (II):

$$P^1-(Sp^1)_{m1}-(Y^1)_{k1}- \qquad II$$

wherein:

$P^1$ is a polymerizable group selected from groups comprising $CH_2$=CW-, $CH_2$=CW-COO-, $CH_2$=C(Ph)-COO-, $CH_2$=CH-COO-Ph-, $CH_2$=CW-CO-NH-, $CH_2$=C(Ph)-CONH-, $CH_2$=C(COOR')-$CH_2$-COO-, $CH_2$=CH-O-, $CH_2$=CH-OOC-, (Ph)-CH=CH-, $CH_3$-CH=N-$(CH_2)_{p1}$-, HO-, HS-, HO$(CH_2)_{p1}$COO-, HS$(CH_2)_{p1}$COO-, HWN-, HOC(O)-, $CH_2$=CH-Ph-$(O)_{p2}$,

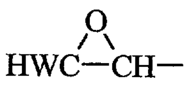
,
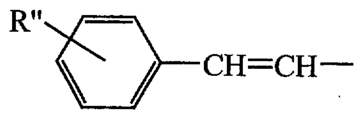
,

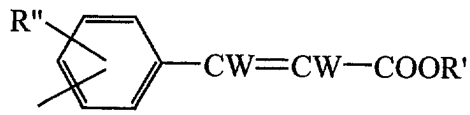
,.

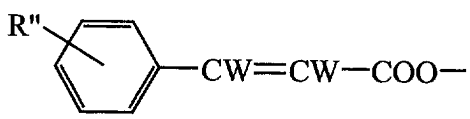
,
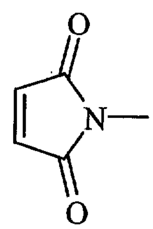
, or
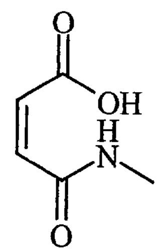
,

wherein:

W represents H, F, Cl, Br or I or a $C_{1-5}$ alkyl group;
$p^1$ is an integer having a value of from 1 to 9;
$p^2$ is an integer having a value of 0 or 1,
R' represents a $C_{1-5}$ alkyl group;
R" represents a $C_{1-5}$ alkyl group, methoxy, cyano, F, Cl, Br or I;
Ph represents phenylene; and
(Ph) represents phenyl.

$Sp^1$ represents an optionally-substituted $C_{1-20}$ alkylene group, in which one or more C-atoms may be replaced by a heteroatom or/and by an optionally substituted aromatic or non-aromatic carbocyclic or heterocyclic 3- to 10-membered ring system or/and it is optionally possible that one or more carbon-carbon single bonds are replaced by carbon-carbon double or triple bond;
m1 is an integer having a value of 0 or 1;
$Y^1$ represents -O-, -S-, -NH-, N($CH_3$)-, -CH(OH)-, -CO-, -$CH_2$(CO)-, -SO-, -$CH_2$(SO)-, -$SO_2$-, -$CH_2$($SO_2$)-, -COO-, -OCO-, -OCO-O-, -S-CO-, -CO-S-, -SOO-, -OSO-, -SOS-, -$CH_2$-$CH_2$-, -$OCH_2$-, -$CH_2$O-, -CH=CH-, or -C≡C-; and
k1 is an integer having a value of 0 or 1.

**7.** Compound according to claim 6, wherein the group $Sp^1$ represents an optionally-substituted $C_{1-20}$ alkylene group, in which one or more C-atoms may be replaced by a heteroatom and/or by an optionally substituted carbocyclic or heterocyclic 3-membered ring system and furthermore one or more carbon-carbon single bond may be replaced by a carbon-carbon double or triple bond.

**8.** Compound according to claims 6 or 7, wherein the $C_{1-20}$ alkylene group of $Sp^1$ may comprise branched or straight chain alkylene groups and may be unsubstituted, mono- or polysubstituted by F, Cl, Br, I or CN.

**9.** Compound according to anyone of claims 6 to 8, wherein one or more of CH2 groups of the $C_{1-20}$ alkylene group of $Sp^1$ maybe replaced, independently, by one or more groups selected from -O-, -S-, -NH-, $-N(CH_3)-$, -CH(OH)-, -CO-, $-CH_2(CO)-$, -SO-, $-CH_2(SO)-$, $-SO_2-$, $-CH_2(SO_2)-$, -COO-, -OCO-, -OCO-O-, -S-CO-, -CO-S-, -SOO-, -OSO-, -SOS-, -C≡C-, $-(CF_2)_{q1}-$ or $-C(W^1)=C(W^2)-$, with the proviso that two oxygen atoms are not directly linked to each other
and $W^1$ and $W^2$ each represent, independently, H, $H-(CH_2)_{q3}-$ or Cl, wherein the integers q1 to q3 each independently represent a number of between 1 and 15.

**10.** Compound according to anyone of claims 1 to 9, wherein the group A represents a group of formula (III):

$$P^2-(Sp^2)_{m1}-Y^2- \qquad III$$

wherein:

$Y^2$ represents -O-, -CO-, -COO-, -OCO-, -C≡C-, or a single bond, especially -O-, -COO-, -OCO- or a single bond;

$Sp^2$ represents a $C_{1-20}$ straight-chain alkylene group, especially ethylene, propylene, butylene, pentylene, hexylene, heptylene, octylene, nonylene, decylene, undecylene, or dodecylene; and

$P^2$ represents a polymerizable group such as : $CH_2=CW^3-$, $CH_2=CW^3-(CO)_{p3}O-$,

0 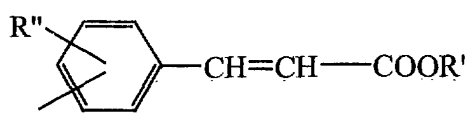

or

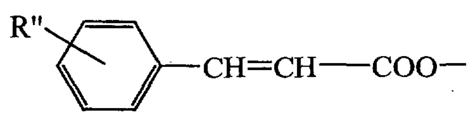

wherein:

$W^3$ represents H, $CH_3$, F, Cl, Br or I; and
p3 is 0 or 1.

**11.** Compound according to claim 10, wherein

$P^2$ represents a polymerizable group $CH_2=CW^3-$ or $CH_2=CW^3-(CO)_{p3}O-$
wherein $W^3$ is H, $CH_3$, F, Cl, Br or I; and
p3 is 0 or 1.

**12.** Compound according to anyone of claims 1 to 11, wherein $Q^1$ is selected from a residue of formula (IV):

$$P^3-Sp^1-(Y^1)_{k1}- \qquad IV$$

wherein:

$P^3$ is hydrogen or a polymerizable group $P^1$ with the meaning given above; and

$Sp^1$, $Y^1$ and k1 have the meaning given above.

**13.** Compound according to anyone of claims 1 to 12, wherein $Q^1$ is selected from a residue of formula (V):

$$P^4\text{-}Sp^2\text{-}Y^2\text{-} \qquad \text{V}$$

wherein:

$P^4$ is hydrogen or a polymerizable group $P^2$ with the meaning given above; and

$Sp^2$ and $Y^2$ have the meaning given above.

**14.** Compound according to anyone of claims 1 to 13, wherein $Q^2$ is selected from a residue of formula (VI):

$$P^5\text{-}(Sp^1)_{m1}\text{-}(Y^1)_{k1}\text{-} \qquad \text{VI}$$

wherein:

$P^5$ is hydrogen; halogen; a polar group such as -CN, and $-NO_2$; or a polymerizable group $P^1$ with the meaning given above and with the proviso that $Q^1$ does not comprise a polymerizable group; and

$Sp^1$, m1, $Y^1$ and $k^1$ have the meaning given above.

**15.** Compound according to anyone of claims 1 to 14, wherein $Q^2$ is selected from a residue of formula (VII):

$$P^5\text{-}(Sp^2\text{-}Y^2)_{m3}\text{-} \qquad \text{VII}$$

wherein:

m3 is an integer having a value of 0 or 1;

$P^5$, $Sp^2$ and $Y^2$ have the meaning given above.

**16.** Compound according to anyone of claims 1 to 15, wherein groups $C^1$ and $C^3$ are selected from:

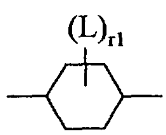 , 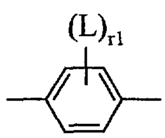 , 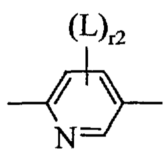 , 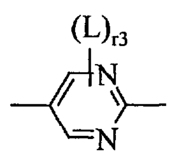 , 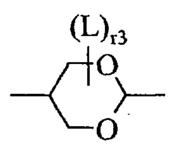 ,

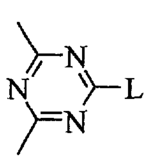 , 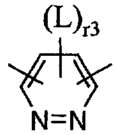 , 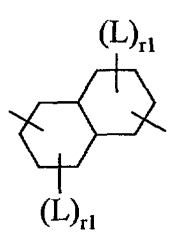 , 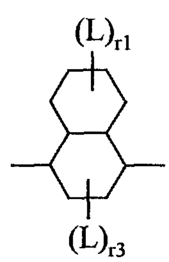 , 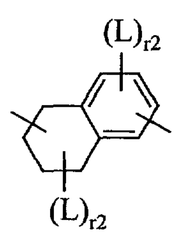 ,

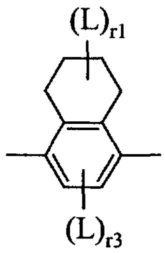 , 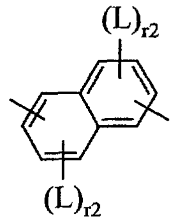 , 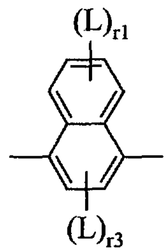 , 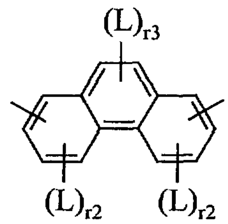 ,

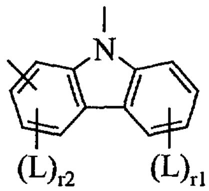 , 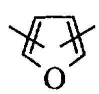 , 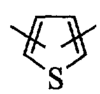 , 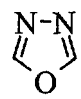 and 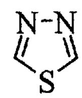

with:

L being $-CH_3$, $-COCH_3$, $-NO_2$, -CN, or halogen
r1 being 0, 1, 2, 3, or 4,
r2 being 0, 1, 2, or 3, and
r3 being 0, 1, or 2.

**17.** Compound according to claim 16, wherein $C^1$ and $C^3$ are selected from the group consisting of optionally-substituted trans-1,4-cyclohexylene, 1,4-phenylene and 2,6- naphthylenediyl.

**18.** Compound according to anyone of claims 1 to 17, wherein group $C^2$ is selected from:

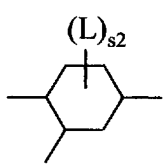 , 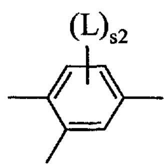 , 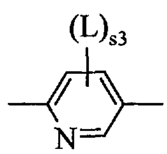 , 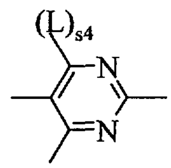 , 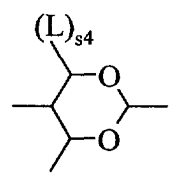 ,

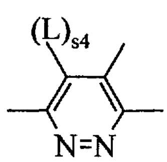 , 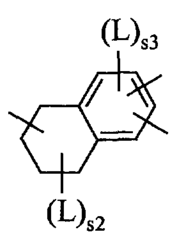 , 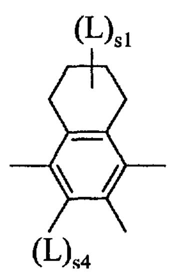 , 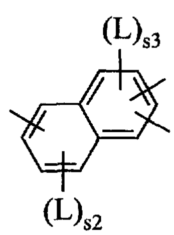 ,

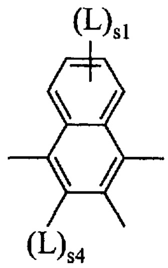 , 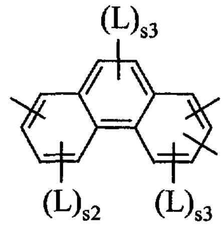 , 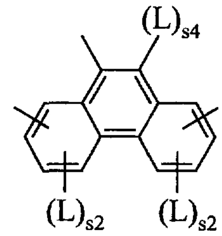 ,

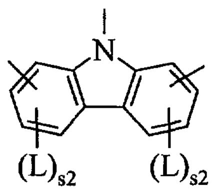 , 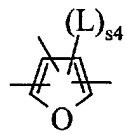 and 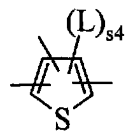

with:

L being -CH3, $-COCH_3$, $-NO_2$, -CN, or halogen,

s1 being 0, 1, 2, 3, or 4,

s2 being 0, 1, 2, or 3,

s3 being 0, 1, or 2 and

s4 being 0 or 1.

**19.** Compound according to claim 18, wherein $C^2$ is selected from the group consisting of optionally-substituted 1,2,4-cyclohexatriyl, 1,2,4-benzenetriyl, 1,2,4-naphthenetriyl, 1,2,6-naphthenetriyl and 2,3,6-naphthenetriyl.

**20.** Compound according to anyone of claims 1 to 19, wherein $X^1$ and $X^2$ are independently selected from the group consisting of -COO-, -OCO-, $-CH_2-CH_2-$, $-CH_2O-$, $-OCH_2-$, -CH=CH-, -C≡C- and a single bond.

**21.** Compound according to claim 20, wherein $X^1$ and $X^2$ are independently selected from the group consisting of of -COO-, -OCO- and a single bond.

**22.** Compound according to anyone of claims 1 to 21, wherein nl and n2 have independently a value from 1 to 3.

**23.** Compound according to anyone of claims 1 to 22, wherein the sum of nl + n2 is 2, 3 or 4.

**24.** Compound according to anyone of claims 1 to 23, wherein

A is a residue selected from the formula (VIII)

$$Y^3-(Sp^2)_{n3}-P^2 \qquad VIII$$

wherein:

$Y^3$ represents -O-, -COO-, -OCO- or a single bond;

n3 is an integer having a value of 0 or 1; and

$Sp^2$ and $P^2$ have the meaning given above;

$Q^1$ is a residue selected from the formula (IX)

$$P^4\text{-}Sp^2\text{-}Y^3 \qquad \text{IX}$$

wherein:
$P^4$, $Sp^2$ and $Y^3$ have the meaning given above;

$Q^2$ is halogen; CN; a polymerizable group $P^2$ with the meaning given above and with the proviso that $Q^1$ does not comprise a polymerizable group; or a residue selected from the formula (X)

$$CH_3\text{-}(Sp^2)_{n3}\text{-}Y^3 \qquad \text{IX}$$

wherein:

$Sp^2$, n3 and $Y^3$ have the meaning given above;

$C^1$ to $C^3$, $X^1$ and $X^2$ have the meaning given above; and

n1 and n2 are integers, wherein the sum of n1 + n2 is 2, 3 or 4.

**25.** Compounds of formula (I) according to claim 1 to 24, which are

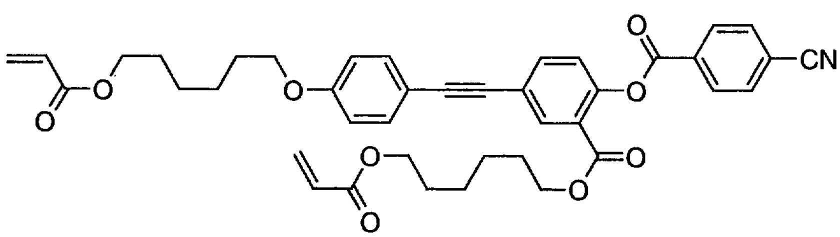

(I. 1)

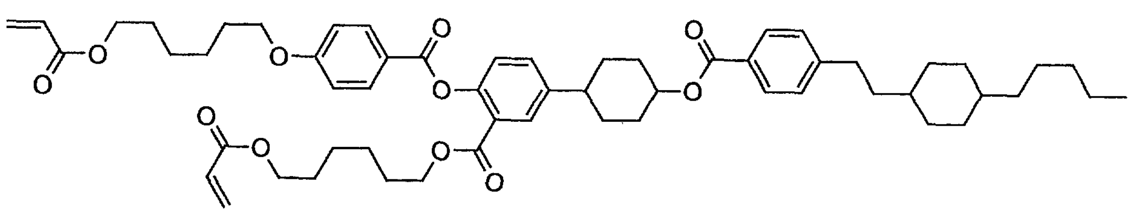

(I. 2)

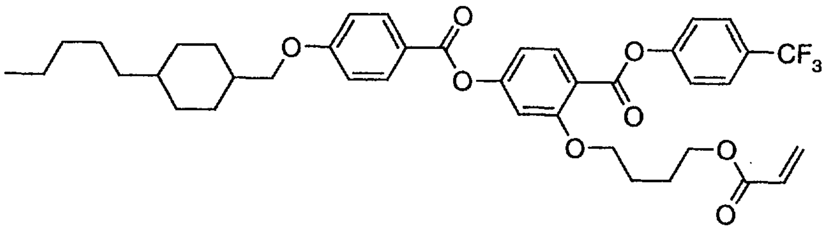

(I. 3)

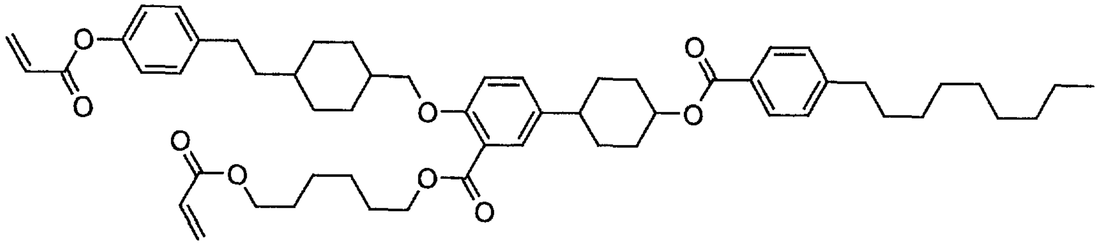

(I. 4)

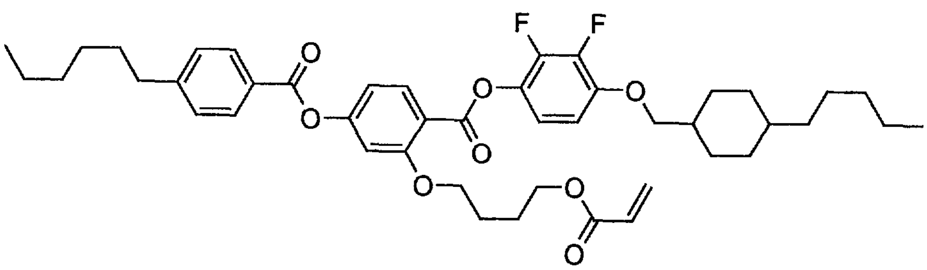

(I. 5)

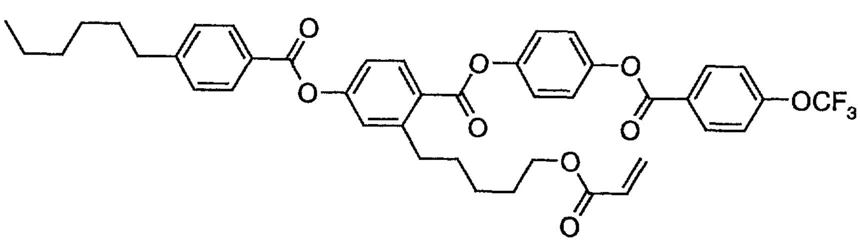

(I. 6)

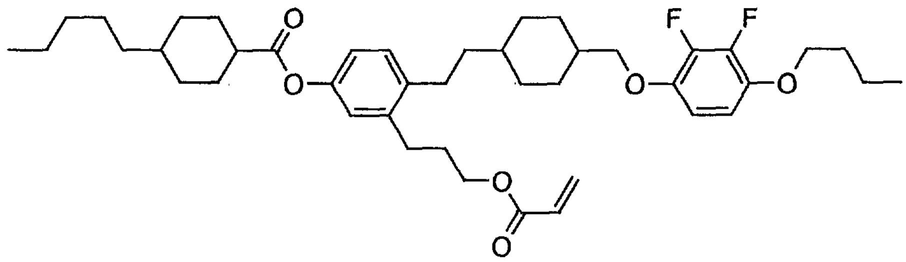

**(I. 7)**

**26.** Use of compounds of formula (I) according to claim 1 to 25 alone or as a component of a liquid crystal mixture.

**27.** A liquid crystalline material comprising a compound of formula (I) according to claim 1 to 25.

**28.** A liquid crystalline material according to claim 27 comprising at least two components.

**29.** A liquid crystalline material according to claims 27 or 28, wherein the additional components are miscible with the compound of formula (I) and may be selected from known mesogenic materials.

**30.** A liquid crystalline material according to anyone of claims 27 to 29, wherein the liquid crystal material is in form of a liquid crystalline mixture, (co)polymer, elastomer, polymer gel or polymer network.

**31.** Use of a liquid crystalline material comprising a compound of formula (I) according to claim 1 to 25 in the manufacture of a liquid crystal polymer.

**32.** A polymer network comprising a compound of formula (I) in crosslinked or polymerized form.

**33.** A polymer network according to claim 32 comprising at least two components, at least one of which is a liquid crystalline compound of formula (I).

**34.** A polymer network according to claims 32 or 33 prepared by polymerization of liquid crystalline materials according to anyone of claims 27 to 30 on a substrate.

**35.** A polymer network according to claims 32 or 33 prepared by copolymerization of a mesogenic mixture comprising:

i) at least one chiral or/and achiral mesogenic polymerizable compound;
ii) at least one compound of formula I; and
iii) an initiator.

**36.** A polymer network according to claim 35, wherein the chiral or achiral mesogenic polymerizable compound is a liquid crystalline compound of formula (I) and/or selected from known mesogenic materials.

**37.** A polymer network according to claims 35 or 36, wherein the chiral or achiral mesogenic polymerizable compound has a thermotropic sequence which includes a nematic phase.

**38.** A polymer network according to anyone of claims 32 to 37 comprising further components.

**39.** A polymer network according to claim 38, wherein the components include additional polymerizable compounds, stabilizers and dyes.

**40.** A polymer network according to claim 39, wherein the additional polymerizable compounds comprise a non-mesogenic compound having at least one polymerizable functional group

**41.** A polymer network according to claim 40, wherein the polymerizable compound is a diacrylate.

**42.** A polymer network according to anyone of claims 35 to 41, wherein the chiral or achiral polymerizable mesogenic compound is present in an amount from 0.01 to 99 % by weight.

**43.** A polymer network according to claim 42, wherein the chiral or achiral polymerizable mesogenic compound is present in an amount from 50 to 95 % by weight.

**44.** A polymer network according to anyone of claims 33 to 43, wherein the liquid crystalline compound of formula (I) may be present in an amount from 0.1 to 100 % by weight.

**45.** A polymer network according to claim 44, wherein the liquid crystalline compound of formula (I) may be present in an amount from 0.1 to 50 % by weight.

**46.** Use of a polymer network according to anyone of claims 32 to 45 in the manufacture of devices such as waveguides, optical gratings, filters, retarders, rotators, piezoelectric cells or thin films exhibiting non-linear optical properties.

**47.** An optical or electro-optical component containing a polymer network comprising a compound of formula (I).

**48.** An optical or electro-optical component according to claim 47 which is a waveguide, an optical grating, a filter, a retarder, a rotator, a piezoelectric cell or a non-linear optical cell or film.

European Patent Office

**PARTIAL EUROPEAN SEARCH REPORT**

which under Rule 45 of the European Patent Convention shall be considered, for the purposes of subsequent proceedings, as the European search report

Application Number
EP 01 81 0929

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | THOMSEN, DONALD L., III ET AL: "Liquid Crystal Elastomers with Mechanical Properties of a Muscle" MACROMOLECULES (2001), 34(17), 5868-5875, 2001, XP002191513 * the whole document * --- | 1-4,6-9, 14-24, 26,27, 30-32 | C07C69/76 C09K19/20 C09K19/30 |
| X | YUSUKE KAWAKAMI ET AL: "SYNTHESIS AND THERMAL TRANSITION OF SIDE-CHAIN LIQUID CRYSTALLINE POLOXETANES HAVING LATERALLY ATTACHED MESOGENIC GROUP" POLYMER INTERNATIONAL, BARKING, GB, vol. 31, no. 1, 1993, pages 35-40, XP000560810 * the whole document * --- | 1-4,6-9, 14-24, 26,27, 30-32 | |
| X | CHIANG, Y.-C. ET AL: "Electro-rheological behavior of liquid crystal polymers (LCPs) dissolved in a nematic solvent: dependence on temperature and LCP structure" POLYMER (2000), 41(11), 4127-4135, 2000, XP002191514 * the whole document * --- -/-- | 1-4,6-9, 14-24, 26,27, 30-32 | TECHNICAL FIELDS SEARCHED (Int.Cl.7) C07C C09K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 27 February 2002 | Puetz, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 01 81 0929

Claim(s) searched incompletely:
1-48

Reason for the limitation of the search:

Present claims 1-48 relate to an extremely large number of possible compounds of formula (I), use of said compounds as a component of a liquid crystal mixture as well as a liquid crystalline material comprising at least one of said compounds and the use of the liquid crystal material in the manufacture of a liquid crystal polymer, polymer networks comprising at least one of compounds of formula (I) in crosslinked or polymerized form and the use of said polymer networks in the manufacture of devices and a electro-optical component containing a polymer network. Support within the meaning of Article 84 EPC and disclosure within the meaning of Article 83 EPC is to be found, however, for only a very small proportion of the compounds, liquid crystal mixtures, polymer networks and devices claimed. In the present case, the claims so lack support, and the application so lacks disclosure, that a meaningful search over the whole of the claimed scope is impossible. Consequently, the search has been carried out for those parts of the claims which appear to be supported and disclosed, namely those parts relating to the compounds and liquid crystal polymer film prepared in the examples 1-7 and a reasonable generalisation thereof.

European Patent Office

# PARTIAL EUROPEAN SEARCH REPORT

Application Number
EP 01 81 0929

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | WO 00 59966 A (ROLIC AG ;SEIBERLE HUBERT (DE); MARCK GUY (FR); MULLER OLIVIER (FR) 12 October 2000 (2000-10-12)<br>* examples 1,2,6 *<br>* claims 1,5,33,35 *<br>----- | 1-4,6-9, 14-24, 26,27, 30-32 | |
| | | | TECHNICAL FIELDS SEARCHED (Int.Cl.7) |

EPO FORM 1503 03.82 (P04C10)

**ANNEX TO THE EUROPEAN SEARCH REPORT ON EUROPEAN PATENT APPLICATION NO.** EP 01 81 0929

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2002

| Patent document cited in search report | | Publication date | Patent family member(s) | | Publication date |
|---|---|---|---|---|---|
| WO 0059966 | A | 12-10-2000 | AU | 3315400 A | 23-10-2000 |
| | | | EP | 1171493 A1 | 16-01-2002 |
| | | | WO | 0059966 A1 | 12-10-2000 |

EPO FORM P0459

For more details about this annex : see Official Journal of the European Patent Office, No. 12/82